# EUROPEAN PATENT APPLICATION

(11) **EP 2 253 629 A1**
(43) Date of publication of application: **24.11.2010**
(21) Application number: 10166182.5
(22) Date of filing: 21.11.2008
(51) Int. Cl.: C07D 403/06, A61K 31/404, A61P 35/00

(54) **Polymorphs of racemic sunitinib malate, compositions containing them and preparation thereof**

(30) Priority: 21.11.2007 US 989560 P; 20.02.2008 US 30167; 02.06.2008 US 58053; 15.04.2008 US 45196; 28.04.2008 US 48467; 28.07.2008 US 84156; 27.02.2008 US 31773; 05.06.2008 US 59088; 03.04.2008 US 42138; 03.06.2008 US 58417; 13.08.2008 US 88554; 24.10.2008 US 108078; 28.04.2008 US 48460; 21.07.2008 US 82405; 01.04.2008 US 41439; 11.08.2008 US 87859; 07.07.2008 US 78650; 12.06.2008 US 61069; 16.06.2008 US 61920; 04.08.2008 US 85991; 30.09.2008 US 101527; 05.06.2008 US 59222
(62) Divisional of application: 08851719.8
(71) Applicant: Teva Pharmaceutical Industries Ltd., 49131 Petah Tiqva (IL)
(72) Inventor: Jegorov, Alexandr, 37316, Dobra Voda (CZ); Vraspir, Pavel, 79501, Rymarov (CZ); Aronhime, Judith, 76217, Rehovot (IL); Gavenda, Ales, 72528, Ostrava, Lhotka (CZ); Angioletti, Paolo, 20020, Lainate (MI) (IT); Macdonald, Peter, Lindsay, 6925, Gentilino (CH); Scarpitta, Francesca, 10015, Ivrea(to) (IT); Villa, Marco, 20125, Milano (IT); Bigatti, Ettore, 20017, Rho (IT); Canavesi, Augusto, 22070, Locate Veresino (co) (IT)
(74) Representative: Eder, Michael

(57) **Abstract**

The invention provides polymorphs of racemic sunitinib malate, processes for the preparation thereof, and pharmaceutical compositions thereof.

## Description

### Related Applications

This application claims the benefit of U.S. Provisional Application Nos. 60/989,560, filed November 21, 2007; 61/030,167, filed February 20, 2008; 61/042,138, filed April 3, 2008; 61/045,196, filed April 15, 2008; 61/048,467, April 28, 2008; 61/058,053, June 2, 2008; 61/061,069, filed June 12, 2008; 61/061,920, filed June 16, 2008; 61/078,650, filed July 7, 2008; 61/082,405, filed July 21, 2008; 61/108,078, filed October 24, 2008; 61/031,773, filed February 27, 2008; 61/041,439, filed April 1, 2008; 61/048,460, filed April 28, 2008; 61/058,417, filed June 3, 2008; 61/059,088, filed June 5, 2008; 61/084,156, filed July 28, 2008; 61/087,859, filed August 11, 2008; 61/101,527, filed September 30, 2008; 61/059,222, filed June 5, 2008; 61/085,991, filed August 4, 2008; 61/088,554, August 13, 2008, each of which is incorporated herein by reference in its entirety.

### Field of the Invention

The invention encompasses Sunitinib hemi-L-malate, polymorphs thereof, polymorphs of racemic sunitinib malate, compositions containing sunitinib base and either L or racemic malic acid, processes for the preparation thereof, and pharmaceutical compositions thereof.

### Background of the Invention

Sunitinib base, N-[2-(diethylamino) ethyl]-5-[(Z)-(5-fluoro-1, 2-dihydro-2-oxo-3*H*-indol-3-ylidine) methyl]-2, 4-dimethyl-1 *H*-pyrrole-3-carboxamide, of the following formula: 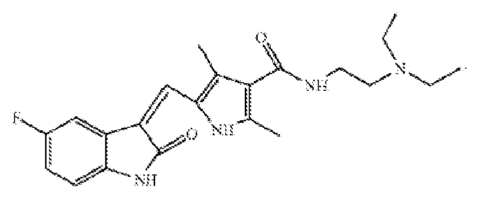 can be used as an intermediate in the preparation of sunitinib salts, such as sunitinib malate of the following formula:

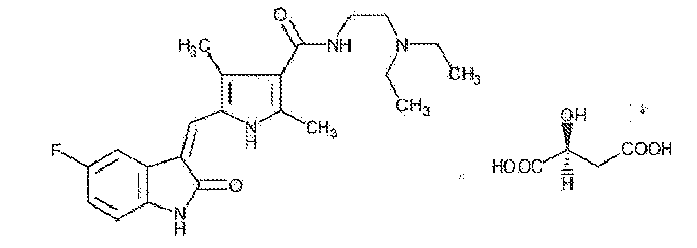

Sunitinib malate is a multi-kinase inhibitor marketed in the United States under the trade name SUTENT^{®} by Pfizer, Inc. SUTENT^{®} is approved by the FDA for the treatment of gastrointestinal stromal tumor after disease progression on or intolerance to imatinib mesylate and for the treatment of advanced renal cell carcinoma. SUTENT^{®} is available as hard-shell capsules containing an amount of sunitinib malate that is equivalent to 12.5 mg, 25 mg, or 50 mg of sunitinib. The capsules contain sunitinib malate together with the inactive ingredients mannitol, croscarmellose sodium, povidone (K-25) and magnesium stearate.

U.S. patent No. 6,573,293 ("'293 patent") refers to the preparation of sunitinib base and salts thereof, as well as the use of these salts. The '293 patent refers to the synthesis of sunitinib base by condensing 5-formyl-2,4-1*H*-pyrrole-3-carboxylic acid (2-diethylaminoethyl)amide with 5-fluoro-1,3-dihydro-indol-2-one in ethanol in the presence of pyrrolidine. See '293 patent, col. 204, II. 33-50 (example 80, alternative synthesis). The sunitinib base thus prepared was isolated from the reaction mixture by filtration, washed with ethanol, slurried in ethanol, isolated from the slurry by filtration, washed with ethanol, and dried under vacuum to give an orange solid. *See id.*

U.S. patent No. 7,119,209 ("'209 patent") also refers to the preparation of sunitinib base. The '209 patent refers to the preparation of sunitinib base by reacting 4-(1H-imidazol-1-ylcarbonyl)-3, 5-dimethyl-1H-pyrrole-2-carbaldehyde, N, N-diethylethylenediamine, 5-fluorooxindole in acetonitrile in the presence of triethylamine. See '209 patent, col. 15, II. 1-36. The sunitinib base thus prepared was isolated from the reaction mixture by filtration, washed with acetonitrile, and dried under vacuum. *See id.*

U.S. Publication No. 2003/0069298 and U.S. Publication No. 2007/0191458 refer to the preparation of sunitinib L-malate, and also disclose two polymorphs thereof.

Polymorphism, the occurrence of different crystal forms, is a property of some molecules and molecular complexes. A single molecule may give rise to a variety of polymorphs having distinct crystal structures and physical properties like melting point, x-ray diffraction pattern, infrared absorption fingerprint, and solid state NMR spectrum. One polymorph may give rise to thermal behavior different from that of another polymorph. Thermal behavior can be measured in the laboratory by such techniques as capillary melting point, thermogravimetric analysis ("TGA"), and differential scanning calorimetry ("DSC"), which have been used to distinguish polymorphic forms.

The difference in the physical properties of different polymorphs results from the orientation and intermolecular interactions of adjacent molecules or complexes in the bulk solid. Accordingly, polymorphs are distinct solids sharing the same molecular formula yet having distinct advantageous physical properties compared to other polymorphs of the same composition or complex.

One of the most important physical properties of pharmaceutical compositions is their solubility in aqueous solution, particularly their solubility in the gastric juices of a patient. For example, where absorption through the gastrointestinal tract is slow, it is often desirable for a drug that is unstable to conditions in the patient's stomach or intestine to dissolve slowly so that it does not accumulate in a deleterious environment. Different polymorphs or polymorphs of the same pharmaceutical compositions can and reportedly do have different aqueous solubilities.

The discovery of new polymorphic forms and solvates of a pharmaceutically useful composition provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic. Therefore, there is a need for additional polymorphs of sunitinib malate.

### Summary of the Invention

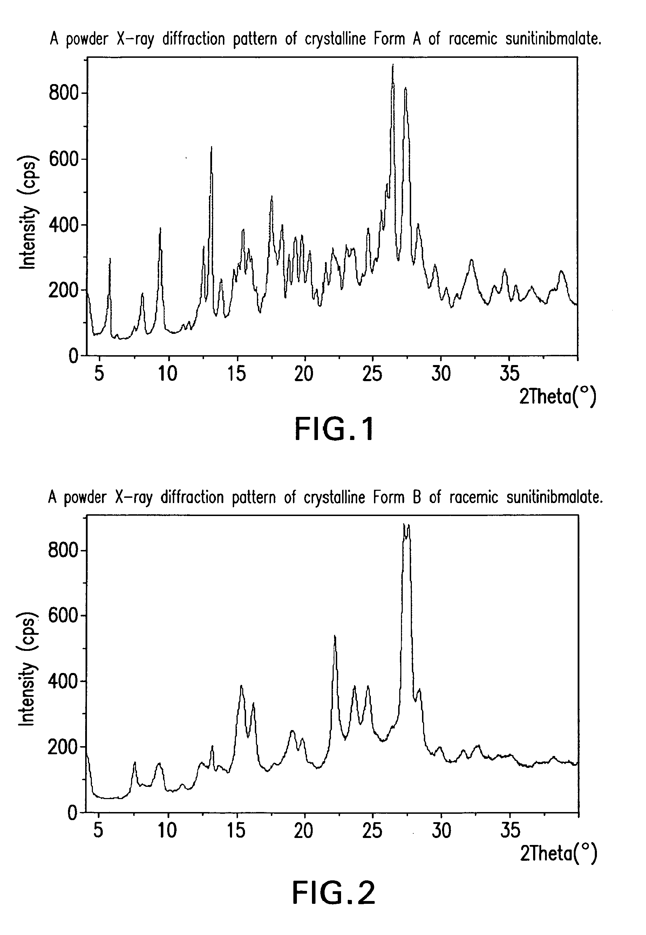

In one embodiment, the invention encompasses a crystalline form of racemic sunitinib malate characterized by data selected from a group consisting of a powder X-ray diffraction ("PXRD") pattern having any 5 peaks at positions selected from the group consisting of: 5.7, 8.0, 9.3, 12.5, 14.7, 15.4, 18.3, 21.5, 24.7 and 27.4 ± 0.2 degrees 2-theta, a PXRD pattern as depicted in Figure 1 and combination thereof.

In one embodiment, the invention encompasses a crystalline form of racemic sunitinib malate characterized by data selected from a group consisting of a PXRD pattern having any 5 peaks at positions selected from the group consisting of: 15.2, 16.1, 22.1, 23.5, 24.5, 27.2 and 27.5 ± 0.2 degrees 2-theta, a PXRD pattern as depicted in Figure 2 and combination thereof.

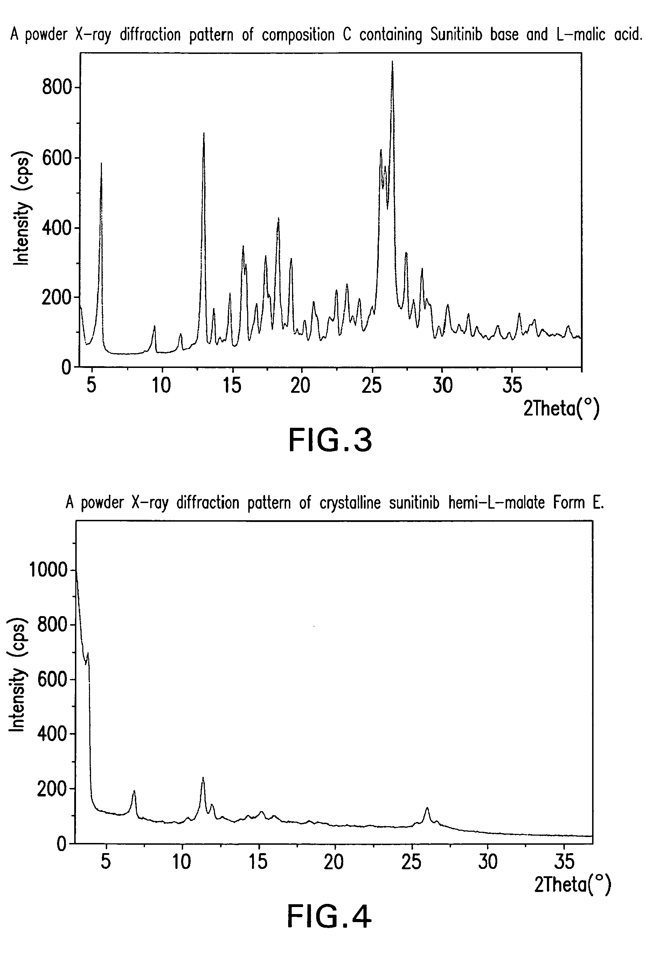

In one embodiment, the invention encompasses a composition containing Sunitinib base and L-malic acid characterized by data selected from a group consisting of a PXRD pattern having any 5 peaks at positions selected from the group consisting of: 5.6, 9.5, 11.4, 13.0, 15.8, 16.8, 17.4, 18.3, 19.2, 20.9, 26.5 and 28.6 ± 0.2 degrees 2-theta, a PXRD pattern as depicted in Figure 3 and combination thereof.

In another embodiment, the invention encompasses Sunitinib hemi-L-malate.

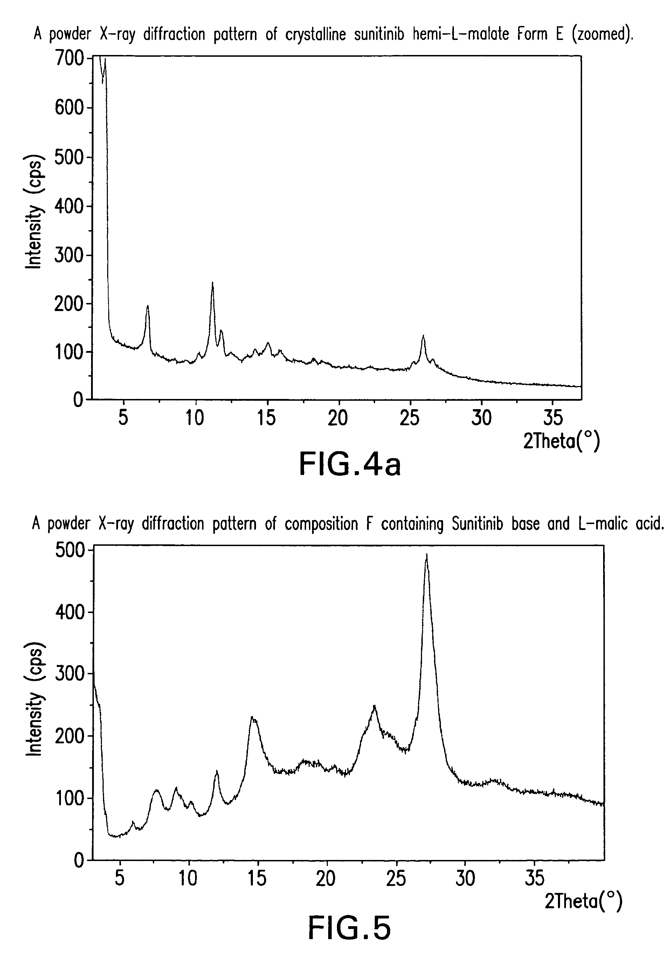

In one embodiment, the invention encompasses a crystalline form of sunitinib hemi-L-malate characterized by data selected from a group consisting of a PXRD pattern having any 5 peaks at positions selected from the group consisting of: 3.7, 6.8, 10.3, 11.3, 11.9, 14.2, 15.1, 15.9, 25.9 and 26.6 ± 0.2 degrees 2-theta, a PXRD pattern as depicted in Figure 4, a PXRD pattern as depicted in Figure 4a and combination thereof.

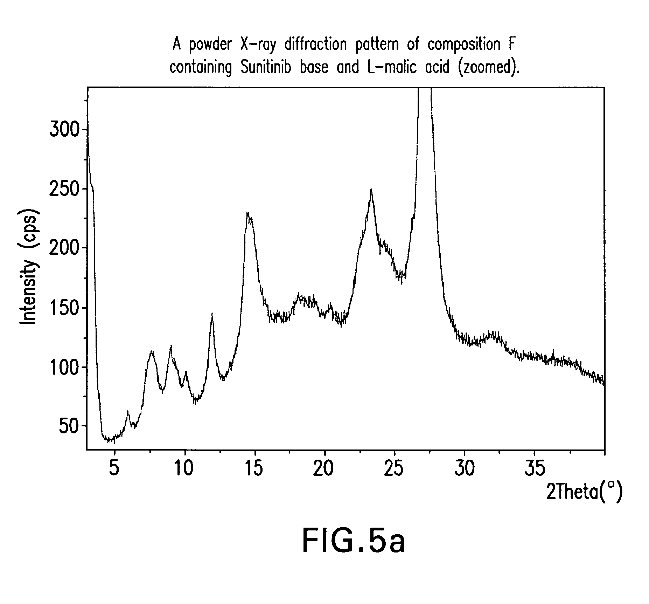

In one embodiment, the invention encompasses a composition containing Sunitinib base and L-malic acid characterized by data selected from a group consisting of a PXRD pattern having any 5 peaks at positions selected from the group consisting of: 6.0, 7.7, 9.1, 10.1, 12.0, 14.5, 23.4 and 27.1 ± 0.2 degrees 2-theta, a PXRD pattern as depicted in Figure 5, a PXRD pattern as depicted in Figure 5a and combination thereof.

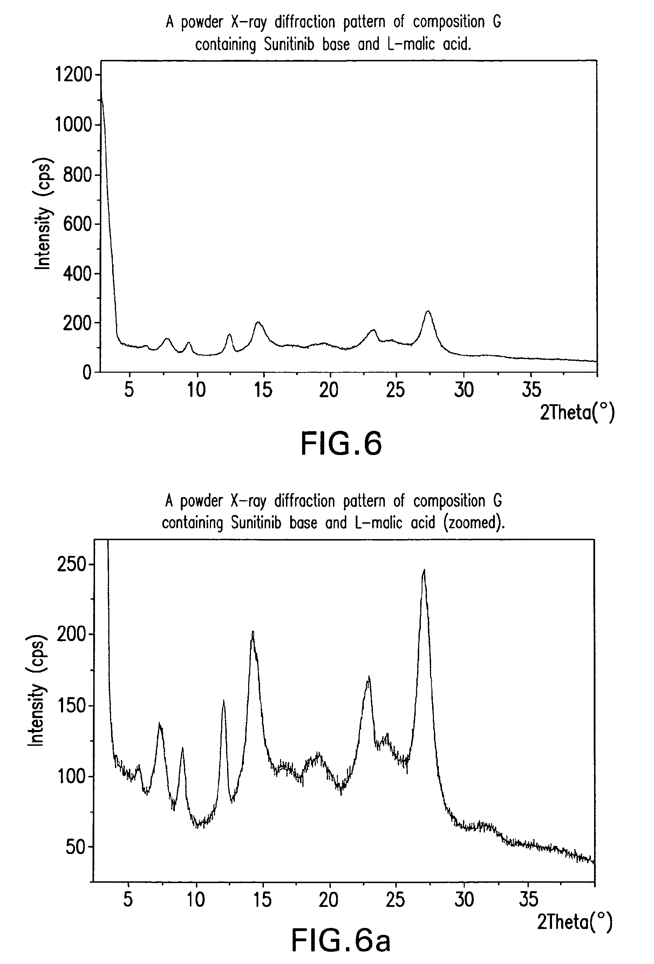

In one embodiment, the invention encompasses a composition containing Sunitinib base and L-malic acid characterized by data selected from a group consisting of a PXRD pattern having any 5 peaks at positions selected from the group consisting of: 6.2, 7.7, 9.3, 12.4, 14.5, 23.2 and 27.4 ± 0.2 degrees 2-theta, a PXRD pattern as depicted in Figure 6, a PXRD pattern as depicted in Figure 6a and combination thereof.

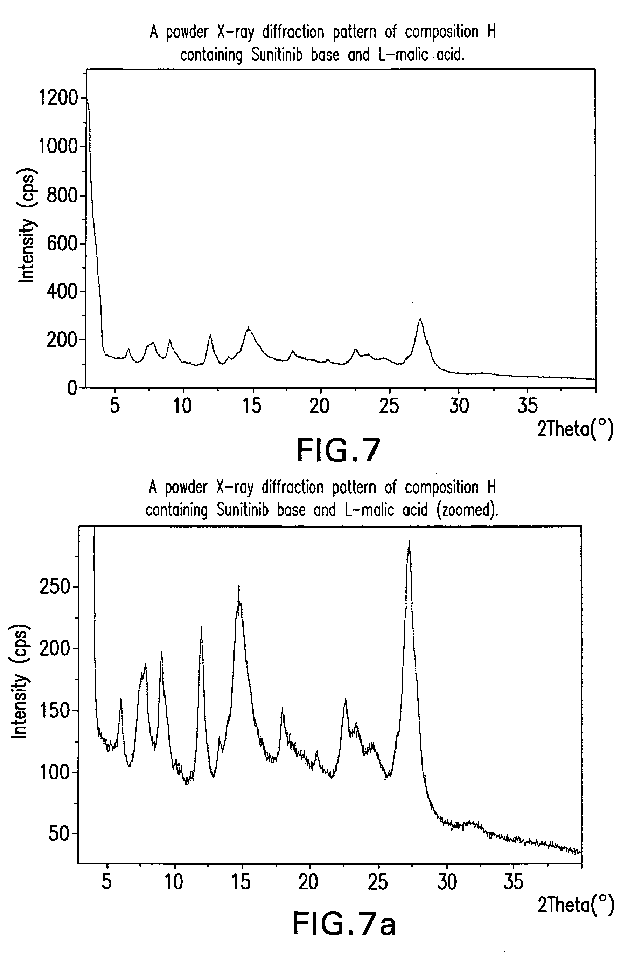

In one embodiment, the invention encompasses a composition containing Sunitinib base and L-malic acid characterized by data selected from a group consisting of a PXRD pattern having any 5 peaks at positions selected from the group consisting of: 6.0, 7.8, 9.0, 12.0, 14.8, 18.0, 22.5 and 27.1 ± 0.2 degrees 2-theta, a PXRD pattern as depicted in Figure 7, a PXRD pattern as depicted in Figure 7a and combination thereof.

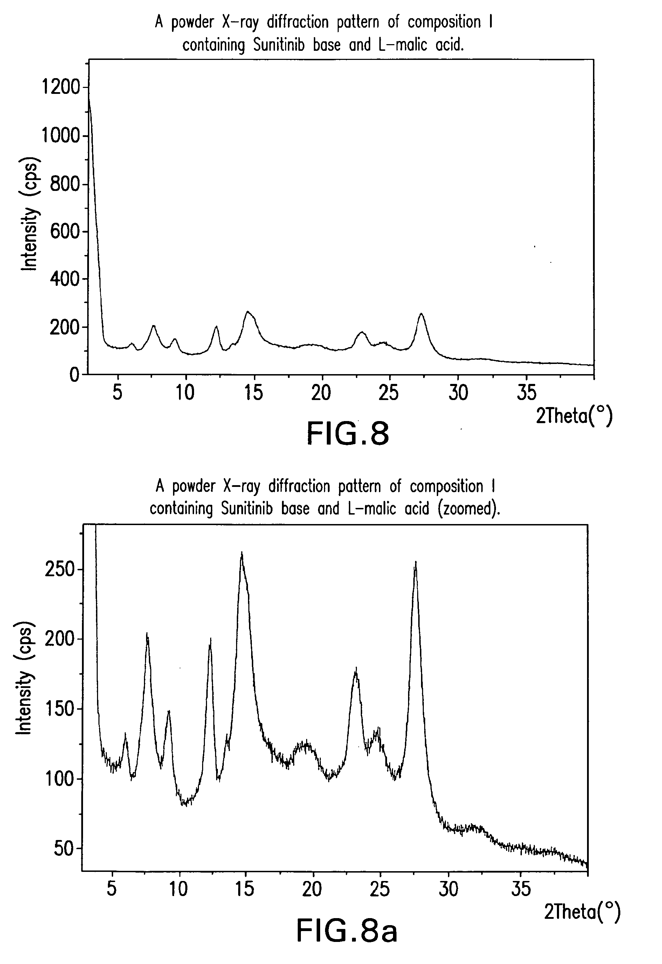

In one embodiment, the invention encompasses a composition containing Sunitinib base and L-malic acid characterized by data selected from a group consisting of a PXRD pattern having any 5 peaks at positions selected from the group consisting of: 6.0, 7.7, 9.2, 12.2, 14.5, 22.9 and 27.3 ± 0.2 degrees 2-theta, a PXRD pattern as depicted in Figure 8, a PXRD pattern as depicted in Figure 8a and combination thereof.

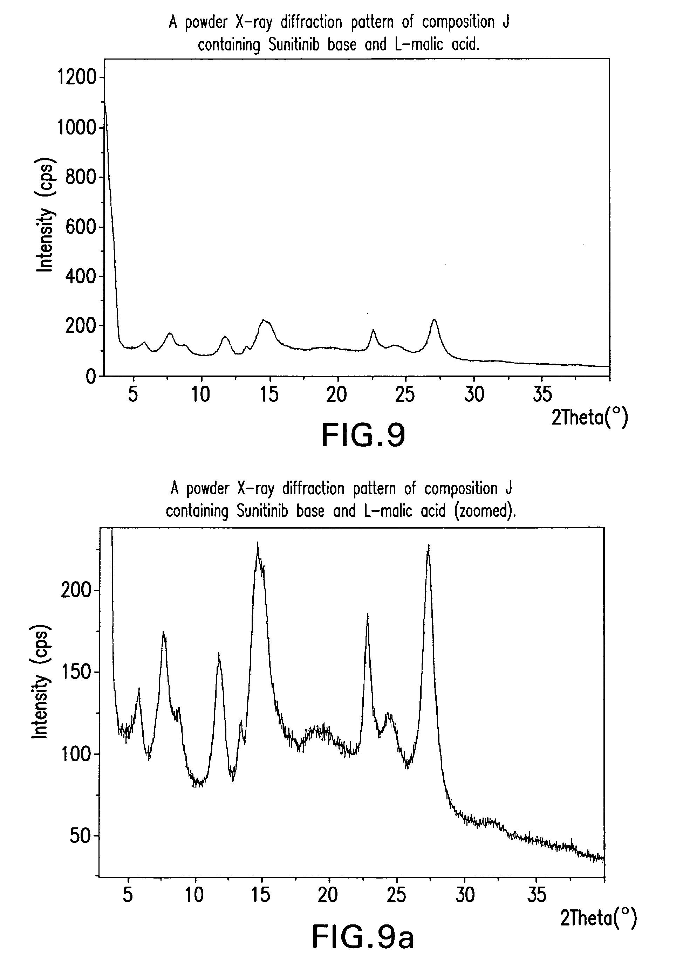

In one embodiment, the invention encompasses a composition containing Sunitinib base and L-malic acid characterized by data selected from a group consisting of a PXRD pattern having any 5 peaks at positions selected from the group consisting of: 5.8, 7.7, 8.7, 11.7, 13.3, 14.5, 22.6 and 27.2 ± 0.2 degrees 2-theta, a PXRD pattern as depicted in Figure 9, a PXRD pattern as depicted in Figure 9a and combination thereof.

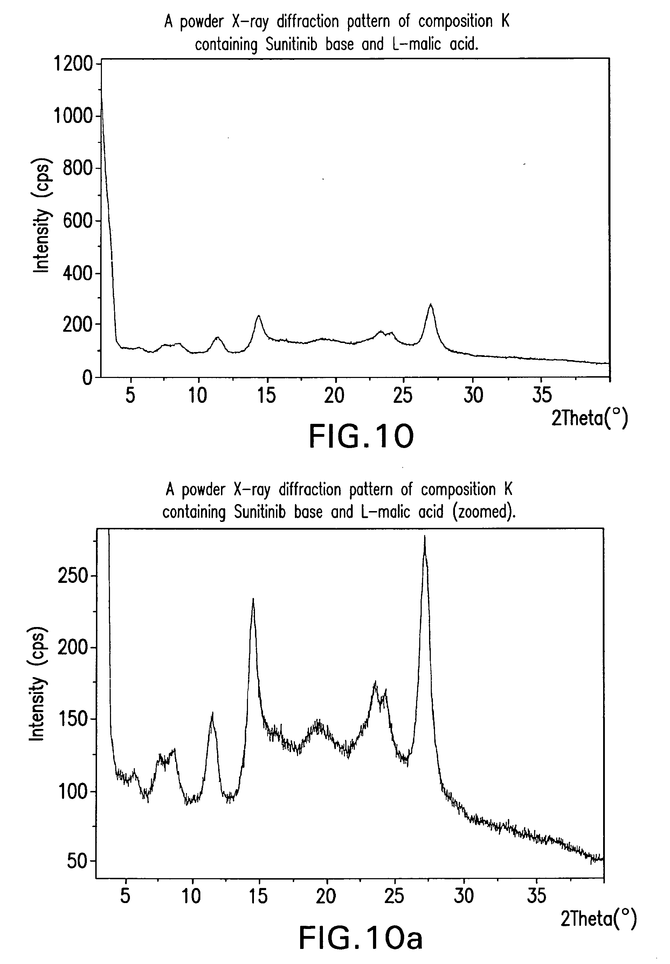

In one embodiment, the invention encompasses a composition containing Sunitinib base and L-malic acid characterized by data selected from a group consisting of a PXRD pattern having peaks at positions selected from the group consisting of: 11.4, 14.4, 23.4, 24.1 and 27.0 ± 0.2 degrees 2-theta, a PXRD pattern as depicted in Figure 10, a PXRD pattern as depicted in Figure 10a and combination thereof.

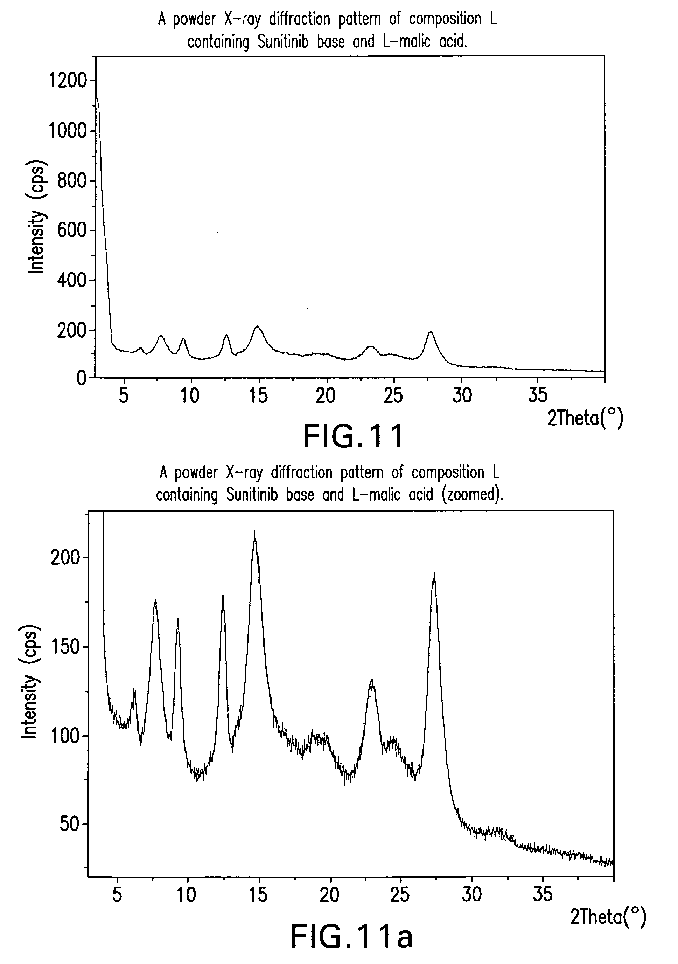

In one embodiment, the invention encompasses a composition containing Sunitinib base and L-malic acid characterized by data selected from a group consisting of a PXRD pattern having any 5 peaks at positions selected from the group consisting of: 6.2, 7.6, 9.3, 12.4, 14.6, 22.9 and 27.4 ± 0.2 degrees 2-theta, a PXRD pattern as depicted in Figure 11, a PXRD pattern as depicted in Figure 11a and combination thereof.

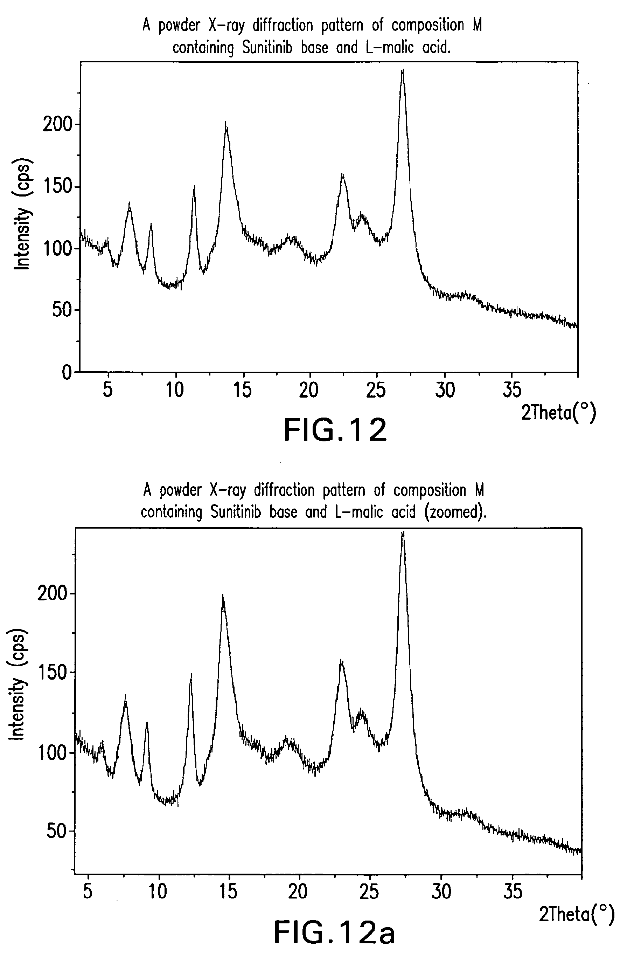

In one embodiment, the invention encompasses a composition containing Sunitinib base and L-malic acid characterized by data selected from a group consisting of a PXRD pattern having any 5 peaks at positions selected from the group consisting of: 6.0, 7.7, 9.2, 12.3, 14.5, 23.0 and 27.3 ± 0.2 degrees 2-theta, a PXRD pattern as depicted in Figure 12, a PXRD pattern as depicted in Figure 12a and combination thereof.

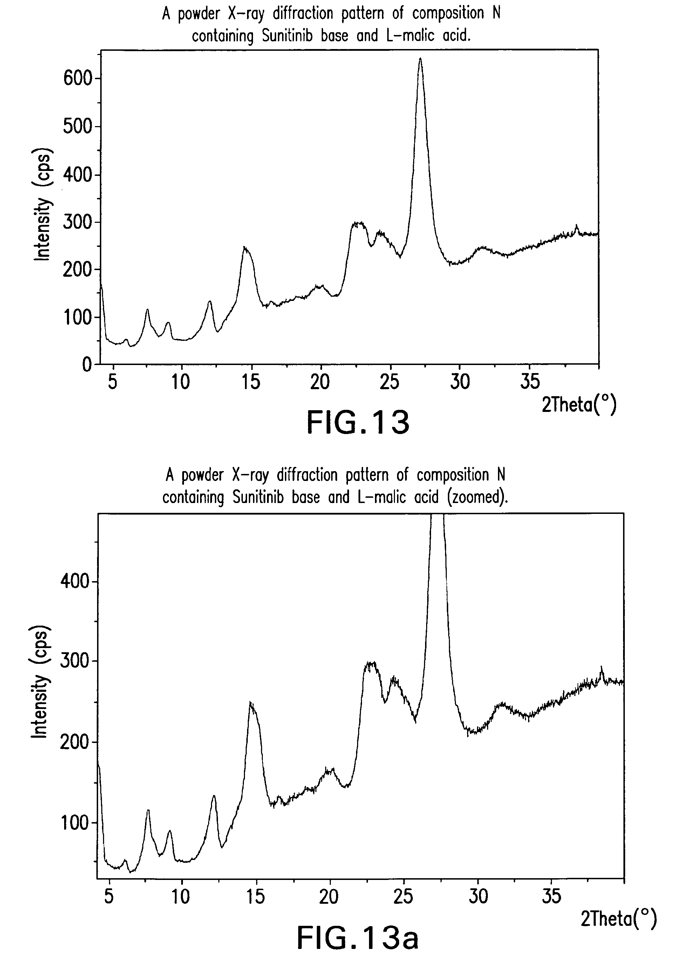

In one embodiment, the invention encompasses a composition containing Sunitinib base and L-malic acid characterized by data selected from a group consisting of a PXRD pattern having any 5 peaks at positions selected from the group consisting of: 6.0, 7.6, 9.2, 12.1, 14.5, 24.2 and 27.2 ± 0.2 degrees 2-theta, a PXRD pattern as depicted in Figure 13, a PXRD pattern as depicted in Figure 13a and combination thereof.

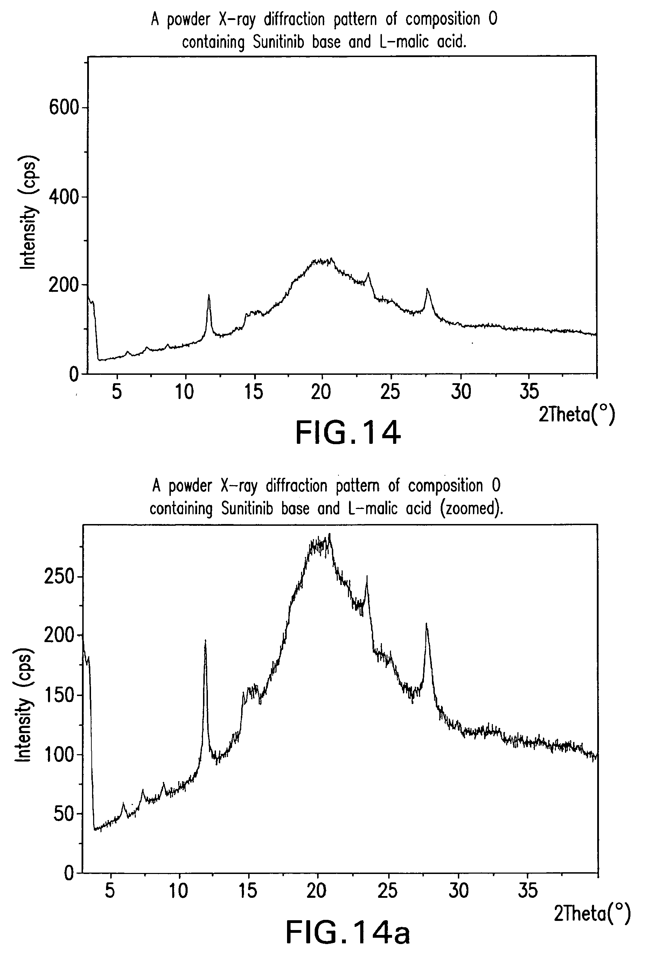

In one embodiment, the invention encompasses a composition containing Sunitinib base and L-malic acid characterized by data selected from a group consisting of a PXRD pattern having any 5 peaks at positions selected from the group consisting of: 6.0, 7.4, 8.9, 11.9, 23.4 and 27.7 ± 0.2 degrees 2-theta, a PXRD pattern as depicted in Figure 14, a PXRD pattern as depicted in Figure 14a and combination thereof.

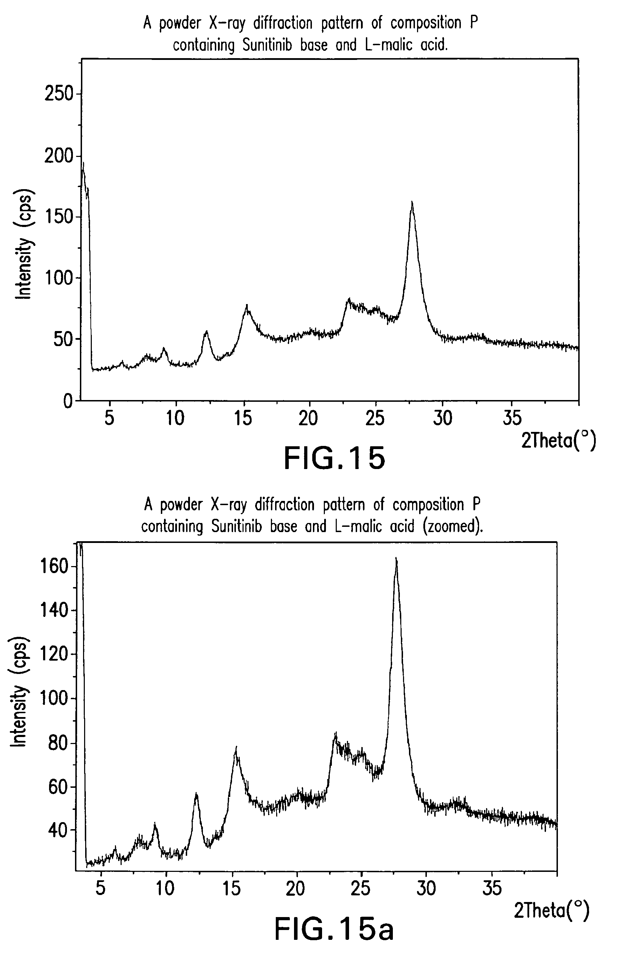

In one embodiment, the invention encompasses a composition containing Sunitinib base and L-malic acid characterized by data selected from a group consisting of a PXRD pattern having any 5 peaks at positions selected from the group consisting of: 6.1, 7.9, 9.2, 12.1, 15.2, 22.9 and 27.7 ± 0.2 degrees 2-theta, a PXRD pattern as depicted in Figure 15, a PXRD pattern as depicted in Figure 15a and combination thereof.

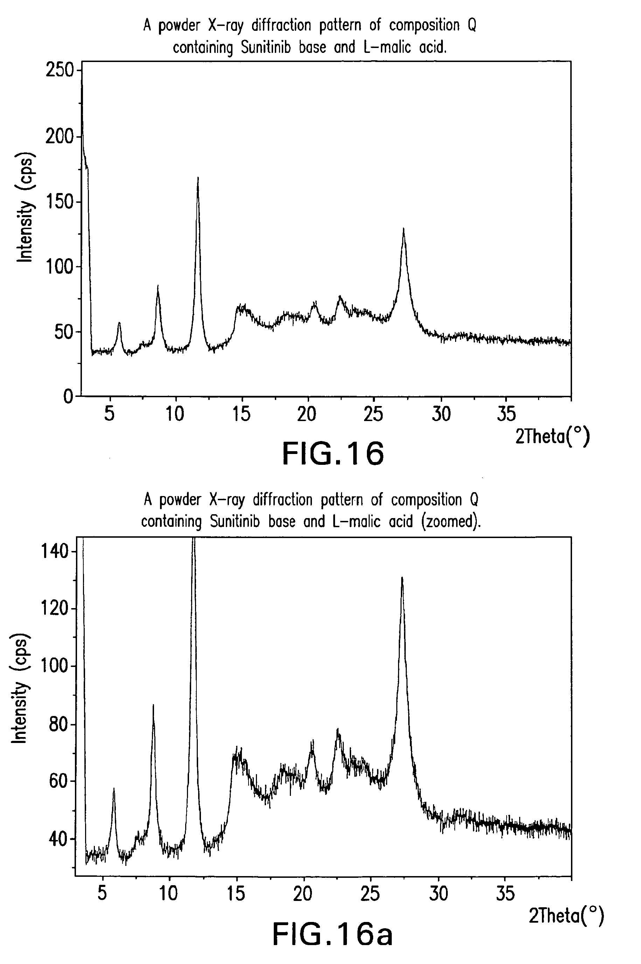

In one embodiment, the invention encompasses a composition containing Sunitinib base and L-malic acid characterized by data selected from a group consisting of a PXRD pattern having any 5 peaks at positions selected from the group consisting of: 5.9, 8.9, 11.8, 20.6, 22.6 and 27.3 ± 0.2 degrees 2-theta, a PXRD pattern as depicted in Figure 16, a PXRD pattern as depicted in Figure 16a and combination thereof.

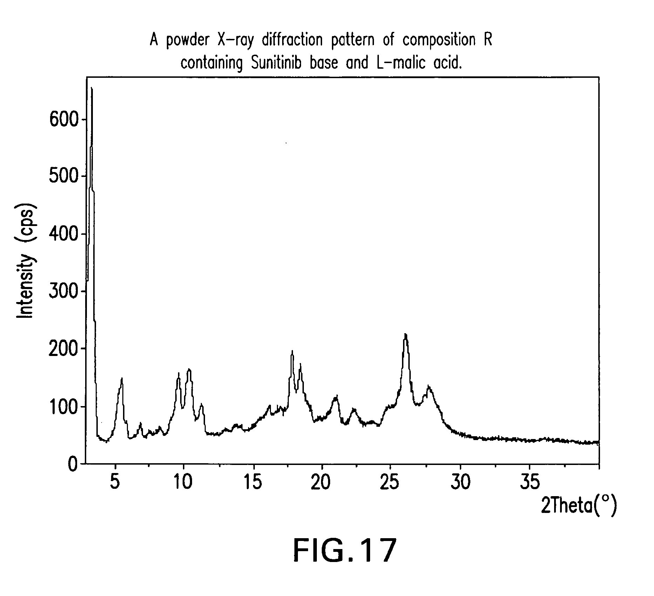

In one embodiment, the invention encompasses a composition containing Sunitinib base and L-malic acid characterized by data selected from a group consisting of a PXRD pattern having any 5 peaks at positions selected from the group consisting of: 3.4, 5.6, 9.6, 10.3, 17.8, 18.4 and 26.0 ± 0.2 degrees 2-theta, a PXRD pattern as depicted in Figure 17 and combination thereof.

In one embodiment, the invention encompasses a crystalline form of sunitinib hemi-L-malate characterized by data selected from a group consisting of: a PXRD pattern any 5 peaks at positions selected from the group consisting of: 5.8, 9.6, 13.9, 15.9, 22.7, 26.6 and 28.7 ± 0.2 degrees 2-theta, a solid-state ¹³C NMR spectrum with signals at about 169.0, 136.0 and 119.5 ± 0.2 ppm, a solid-state ¹³C NMR spectrum having chemical shifts differences between the signal exhibiting the lowest chemical shift and another in the chemical shift range of 100 to 180 ppm of about 66.3, 33.3, 16.8 ± 0.1 ppm, a PXRD pattern as depicted in Figure 18; a ¹³C NMR spectrum depicted in Figure 19, and a solid-state ¹³C NMR spectrum depicted in Figure 20 and combination thereof. This form can be designated as form U.

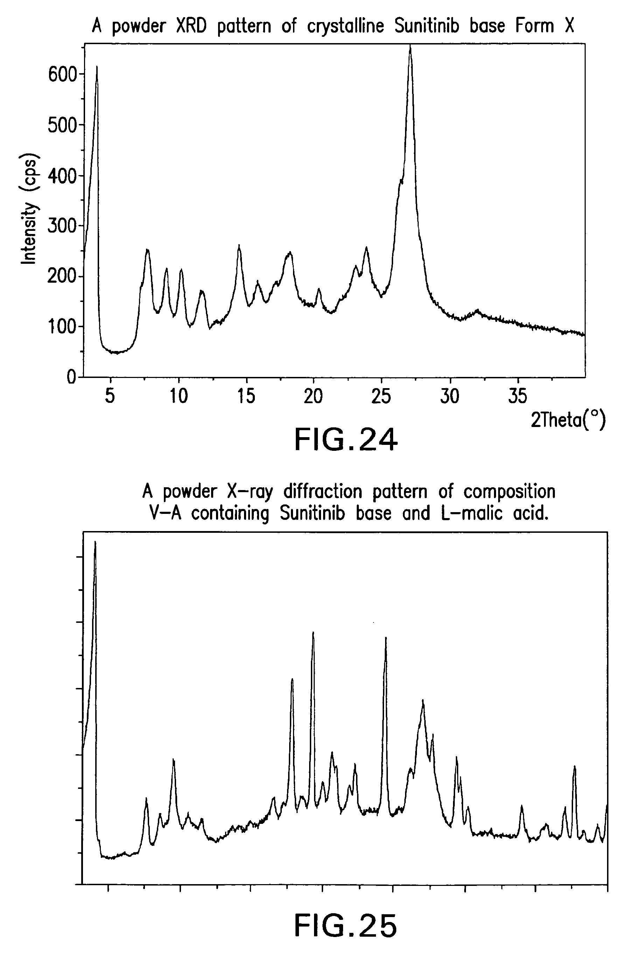

In one embodiment, the invention encompasses a composition containing Sunitinib base and L-malic acid, characterized by data selected from a group consisting of a PXRD pattern having any 5 peaks at about: 8.5, 9.3, 16.5, 17.8, 20.9 and 29.7 ± 0.2 degrees 2-theta, a PXRD pattern as depicted in Figure 25 and combination thereof.

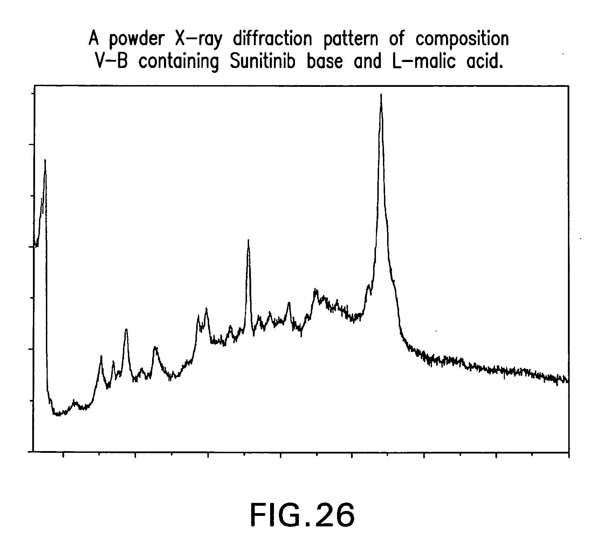

In one embodiment, the present invention encompasses a composition containing Sunitinib base and L-malic acid, characterized by data selected from a group consisting of a PXRD pattern having any 5 peaks at positions selected from the group consisting of: 3.8, 14.3, 14.9, 17.8 and 27.0 ± 0.2 degrees 2-theta, a PXRD pattern as depicted in Figure 26 and combination thereof.

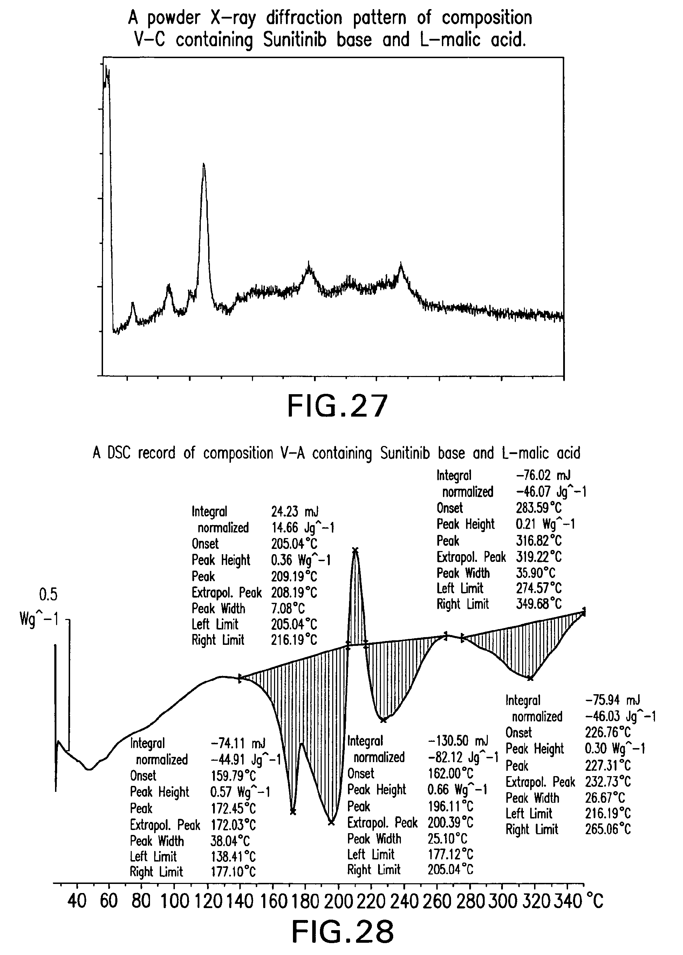

In one embodiment, the present invention encompasses a composition containing Sunitinib base and L-malic acid, characterized by data selected from a group consisting of a PXRD pattern having any 5 peaks at positions selected from the group consisting of: 5.5, 8.4, 11.1, 19.5 and 26.9 ± 0.2 degrees 2-theta, a PXRD pattern as depicted in Figure 27 and combination thereof.

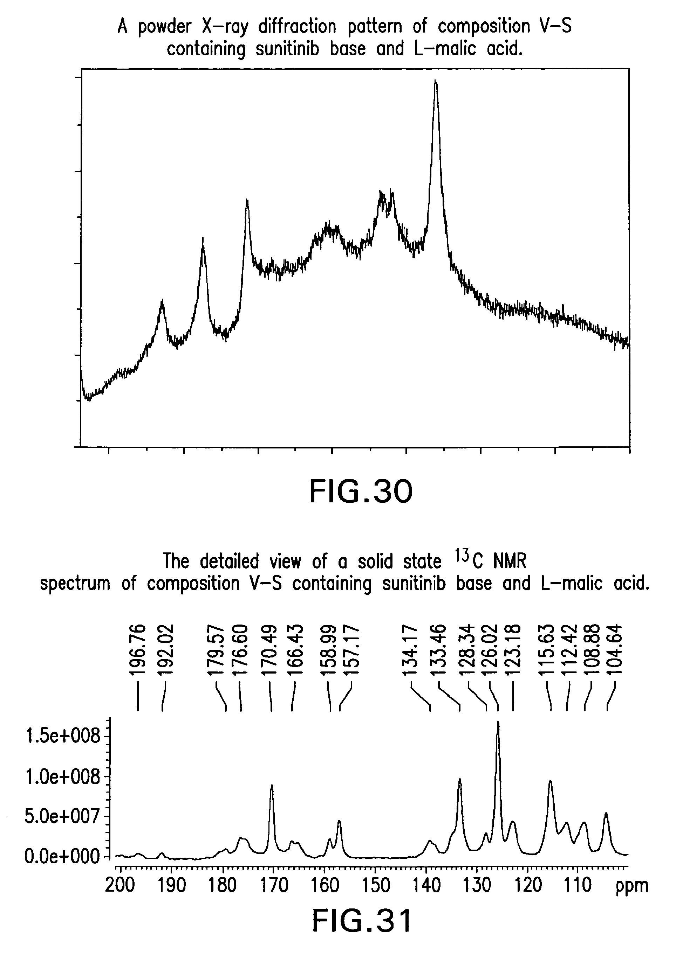

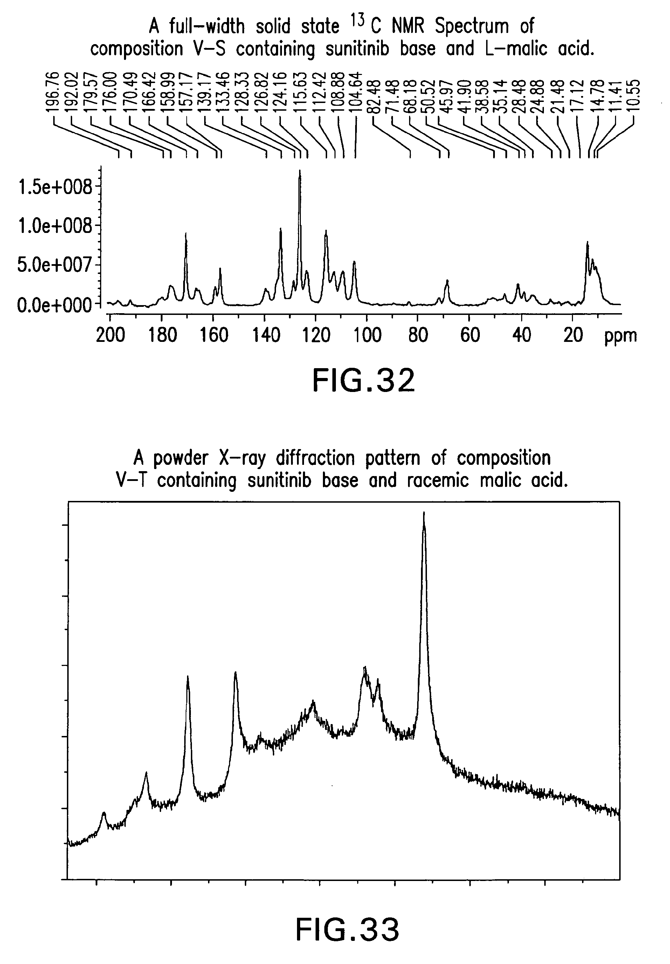

In one embodiment, the invention encompasses a composition containing Sunitinib base and L-malic acid, characterized by data selected from a group consisting of: a PXRD pattern having any 5 peaks at positions selected from the group consisting of 8.5, 11.3, 23.2, 24.0 and 26.9 ± 0.2 degrees 2-theta, a solid-state ¹³C NMR spectrum with peaks at about 170.5, 157.2 and 115.6 ± 0.2 ppm, a solid-state ¹³C NMR spectrum having chemical shifts differences between the signal exhibiting the lowest chemical shift and another in the chemical shift range of 100 to 180 ppm of about 65.9, 52.6 and 11.0 ± 0.1 ppm, a PXRD pattern as depicted in Figure 30, a solid-state ¹³C NMR spectrum depicted in Figure 31, a solid-state ¹³C NMR spectrum depicted in Figure 32 and combination thereof.

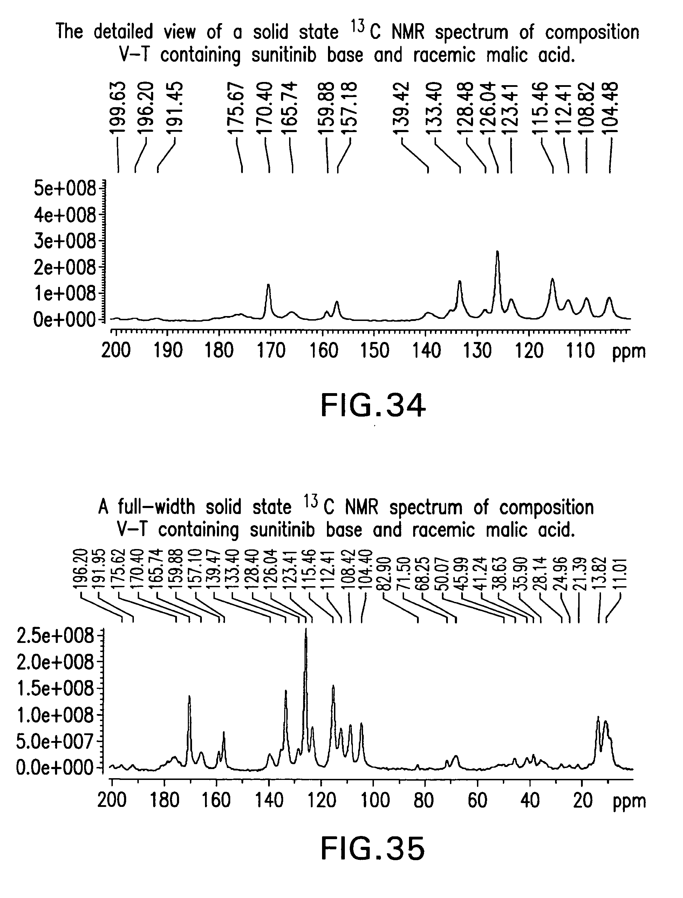

In one embodiment, the invention encompasses a composition containing Sunitinib base and racemic malic acid, characterized by data selected from a group consisting of: a PXRD pattern having any 5 peaks at positions selected from the group consisting of: 5.5, 8.3, 11.1, 14.2, 22.9, 23.8 and 26.8 ± 0.2 degrees 2-theta, a solid-state ¹³C NMR spectrum with signals at about 170.4, 157.2 and 115.5 ± 0.2 ppm, a solid-state ¹³C NMR spectrum having chemical shifts differences between the signal exhibiting the lowest chemical shift and another in the chemical shift range of 100 to 180 ppm of about 65.9, 52.7 and 11.0 ± 0.1 ppm, a PXRD pattern as depicted in Figure 33, a solid-state ¹³C NMR spectrum depicted in Figure 34, a solid-state ¹³C NMR spectrum depicted in Figure 35 and combination thereof.

In yet another embodiment, the invention encompasses a process for preparing pharmaceutical composition comprising at least one of the above-described polymorphs of racemic sunitinib malate, sunitinib hemi-L-malate or of the above-described compositions containing Sunitinib base and either L or racemic malic acid, comprising combining at least one of the above-described polymorphs of racemic sunitinib malate, sunitinib hemi-L-malate or of the above-described compositions containing Sunitinib base and either L or racemic malic acid, and at least one pharmaceutically acceptable excipient.

In yet another embodiment, the invention encompasses a pharmaceutical composition comprising at least one of the above-described polymorphs of racemic sunitinib malate, sunitinib hemi-L-malate or of the above-described compositions containing Sunitinib base and L or racemic malic acid, prepared according to the processes disclosed herein and at least one pharmaceutically acceptable excipient.

In yet another embodiment, the invention encompasses a pharmaceutical composition comprising at least one of the above-described polymorphs of racemic sunitinib malate, sunitinib hemi-L-malate or of the above-described compositions containing Sunitinib base and L or racemic malic acid, and at least one pharmaceutically acceptable excipient.

In another embodiment, the invention encompasses a method of treating advanced renal cell carcinoma comprising administering to a patient in need thereof a pharmaceutical composition comprising at least one of the above-described polymorphs of racemic sunitinib malate, sunitinib hemi-L-malate or of the above compositions containing Sunitinib base and either L or racemic malic acid and at least one pharmaceutically acceptable excipient.

One embodiment of the invention provides the use of the above polymorphs of racemic Sunitinib malate, sunitinib hemi-L-malate or of the above-described compositions containing Sunitinib base and L or racemic malic acid of the present invention for the manufacture of a medicament for the treatment of gastrointenstinal stromal tumor or for the treatment of advanced renal cell

One embodiment of the invention provides the use of the above polymorphs of racemic Sunitinib malate, sunitinib hemi-L-malate or of the above-described compositions containing Sunitinib base and L or racemic malic acid of the present invention as a medicament for the treatment of gastrointenstinal stromal tumor or for the treatment of advanced renal cell carcinoma.

### Brief Description of the Drawings

Figure 1 illustrates a powder X-ray diffraction pattern of crystalline racemic sunitinib malate Form A.
Figure 2 illustrates a powder X-ray diffraction pattern of crystalline racemic sunitinib malate Form B.
Figure 3 illustrates a powder X-ray diffraction pattern of composition C containing Sunitinib base and L-malic acid.
Figure 4 illustrates a powder X-ray diffraction pattern of crystalline sunitinib hemi-L-malate Form E.
Figure 4a illustrates a powder X-ray diffraction pattern of crystalline sunitinib hemi-L-malate Form E (zoomed).
Figure 5 illustrates a powder X-ray diffraction pattern of composition F containing Sunitinib base and L-malic acid.
Figure 5a illustrates a powder X-ray diffraction pattern of composition F containing Sunitinib base and L-malic acid (zoomed).
Figure 6 illustrates a powder X-ray diffraction pattern of composition G containing Sunitinib base and L-malic acid.
Figure 6a illustrates a powder X-ray diffraction pattern of composition G containing Sunitinib base and L-malic acid (zoomed).
Figure 7 illustrates a powder X-ray diffraction pattern of composition H containing Sunitinib base and L-malic acid.
Figure 7a illustrates a powder X-ray diffraction pattern of composition H containing Sunitinib base and L-malic acid (zoomed).
Figure 8 illustrates a powder X-ray diffraction pattern of composition I containing Sunitinib base and L-malic acid.
Figure 8a illustrates a powder X-ray diffraction pattern of composition I containing Sunitinib base and L-malic acid (zoomed).
Figure 9 illustrates a powder X-ray diffraction pattern of composition J containing Sunitinib base and L-malic acid.
Figure 9a illustrates a powder X-ray diffraction pattern of composition J containing Sunitinib base and L-malic acid (zoomed).
Figure 10 illustrates a powder X-ray diffraction pattern of composition K containing Sunitinib base and L-malic acid.
Figure 10a illustrates a powder X-ray diffraction pattern of composition K containing Sunitinib base and L-malic acid (zoomed).
Figure 11 illustrates a powder X-ray diffraction pattern of composition L containing Sunitinib base and L-malic acid.
Figure 11a illustrates a powder X-ray diffraction pattern of composition L containing Sunitinib base and L-malic acid (zoomed).
Figure 12 illustrates a powder X-ray diffraction pattern of composition M containing Sunitinib base and L-malic acid.
Figure 12a illustrates a powder X-ray diffraction pattern of composition M containing Sunitinib base and L-malic acid (zoomed).
Figure 13 illustrates a powder X-ray diffraction pattern of composition N containing Sunitinib base and L-malic acid.
Figure 13a illustrates a powder X-ray diffraction pattern of composition N containing Sunitinib base and L-malic acid (zoomed).
Figure 14 illustrates a powder X-ray diffraction pattern of composition O containing Sunitinib base and L-malic acid.
Figure 14a illustrates a powder X-ray diffraction pattern of composition O containing Sunitinib base and L-malic acid (zoomed).
Figure 15 illustrates a powder X-ray diffraction pattern of composition P containing Sunitinib base and L-malic acid.
Figure 15a illustrates a powder X-ray diffraction pattern of composition P containing Sunitinib base and L-malic acid (zoomed).
Figure 16 illustrates a powder X-ray diffraction pattern of composition Q containing Sunitinib base and L-malic acid.
Figure 16a illustrates a powder X-ray diffraction pattern of composition Q containing Sunitinib base and L-malic acid (zoomed).
Figure 17 illustrates a powder X-ray diffraction pattern of composition R containing Sunitinib base and L-malic acid.

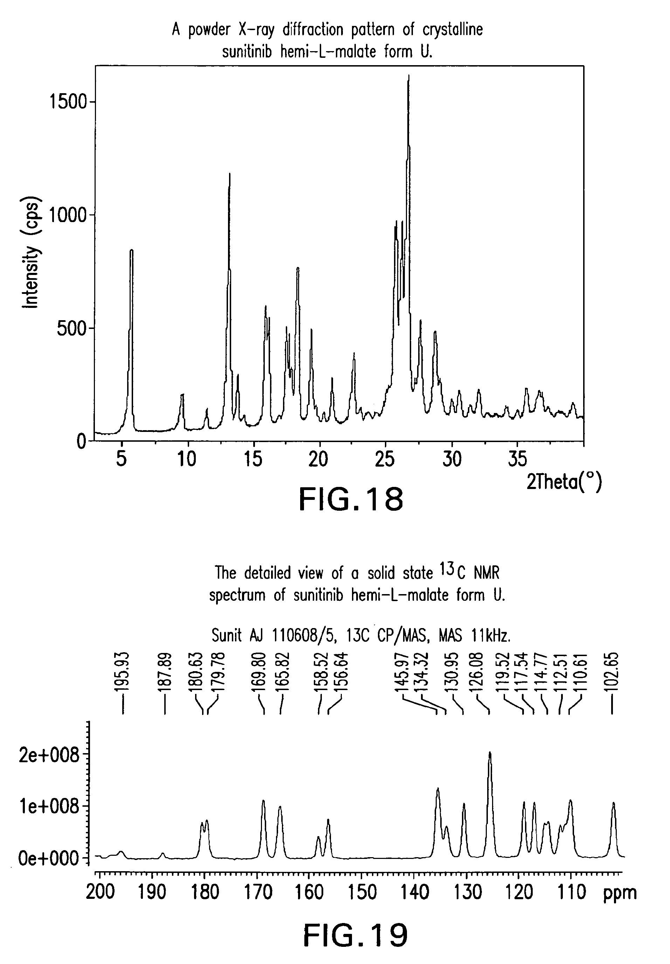

Figure 18 illustrates a powder X-ray diffraction pattern of crystalline sunitinib hemi-L-malate form U.
Figure 19 illustrates a detailed view of a solid state ¹³C NMR spectrum of sunitinib hemi-L-malate form U.

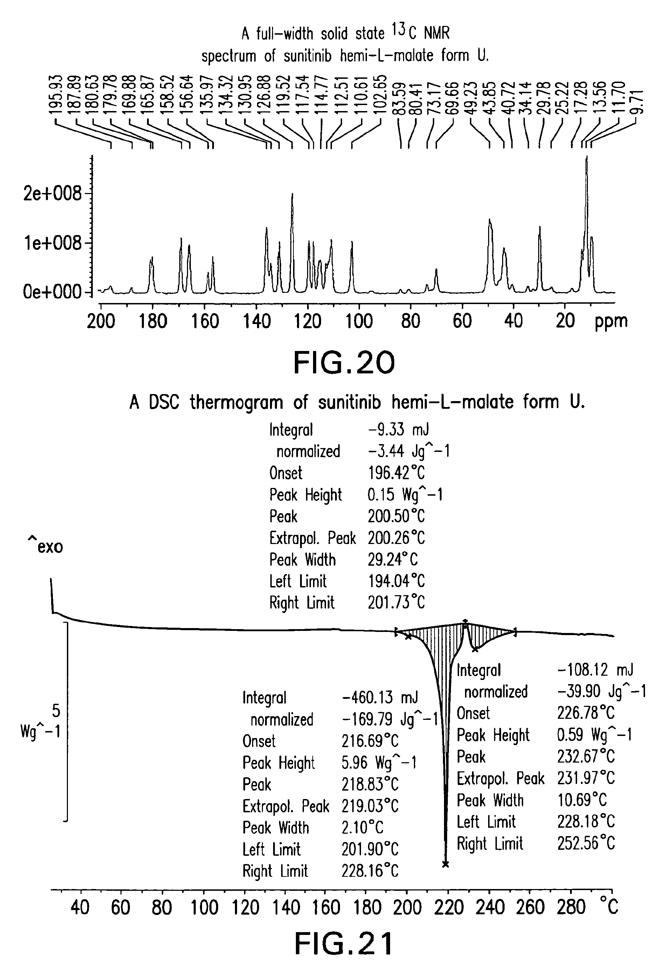

Figure 20 illustrates a full-width solid state ¹³C NMR spectrum of sunitinib hemi-L-malate form U.
Figure 21 illustrates a DSC thermogram of of sunitinib hemi-L-malate form U.

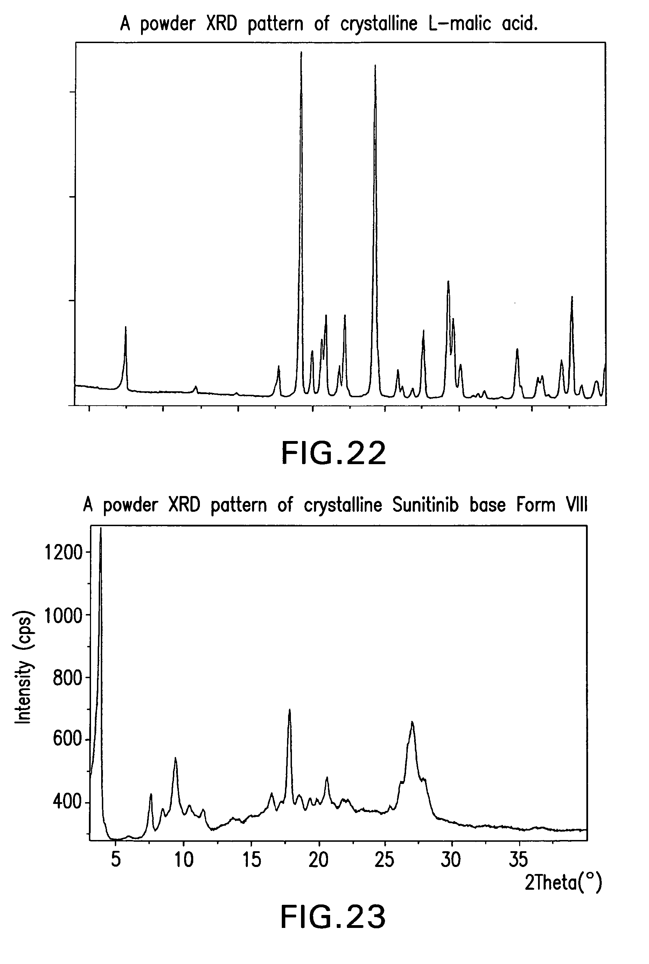

Figure 22 illustrates a powder X-ray diffraction pattern of crystalline L-malic acid.
Figure 23 illustrates a powder X-ray diffraction pattern of crystalline Sunitinib base form VIII.
Figure 24 illustrates A powder XRD pattern of crystalline Sunitinib base Form X
Figure 25 illustrates a powder X-ray diffraction pattern of composition V-A containing Sunitinib base and L-malic acid.
Figure 26 illustrates a powder X-ray diffraction pattern of composition V-B containing Sunitinib base and L-malic acid.
Figure 27 illustrates a powder X-ray diffraction pattern of composition V-C containing Sunitinib base and L-malic acid.
Figure 28 shows a DSC record of composition V-A containing Sunitinib base and L-malic acid.

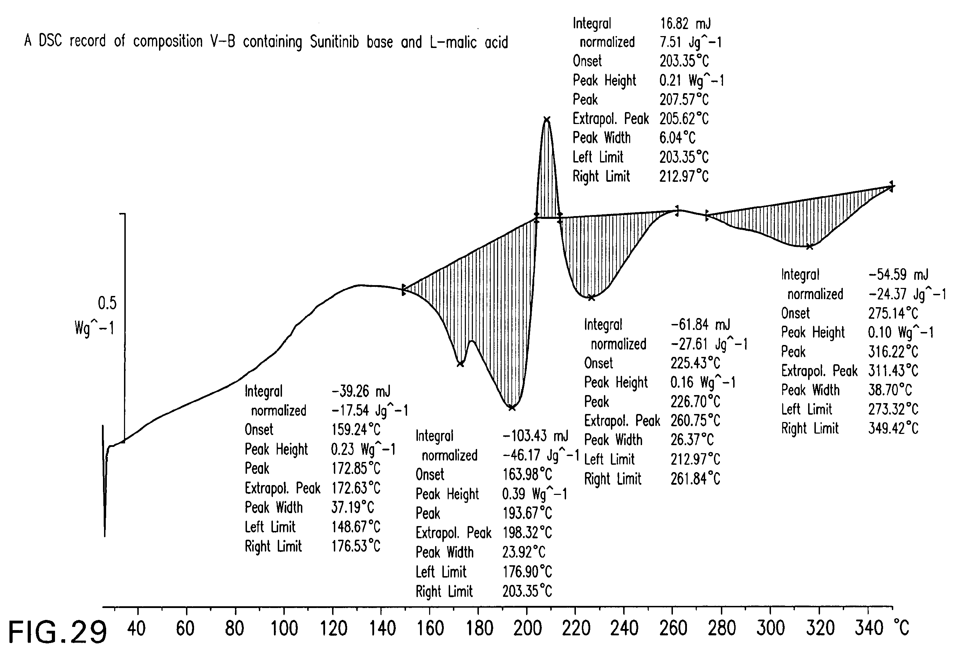

Figure 29 shows a DSC record of composition V-B containing Sunitinib base and L-malic acid.
Figure 30 illustrates a powder X-ray diffraction pattern of composition V-S containing Sunitinib base and L-malic acid.
Figure 31 illustrates a detailed view of a solid state ¹³C NMR spectrum of composition V-S containing Sunitinib base and L-malic acid.
Figure 32 illustrates a full-width solid state ¹³C NMR spectrum of composition V-S containing Sunitinib base and L-malic acid.
Figure 33 illustrates a powder X-ray diffraction pattern of composition V-T of Sunitinib base and racemic malic acid.
Figure 34 illustrates a detailed view of a solid state ¹³C NMR spectrum of composition V-T of Sunitinib base and racemic malic acid.
Figure 35 illustrates a full-width solid state ¹³C NMR spectrum of composition V-T of Sunitinib base and racemic malic acid.

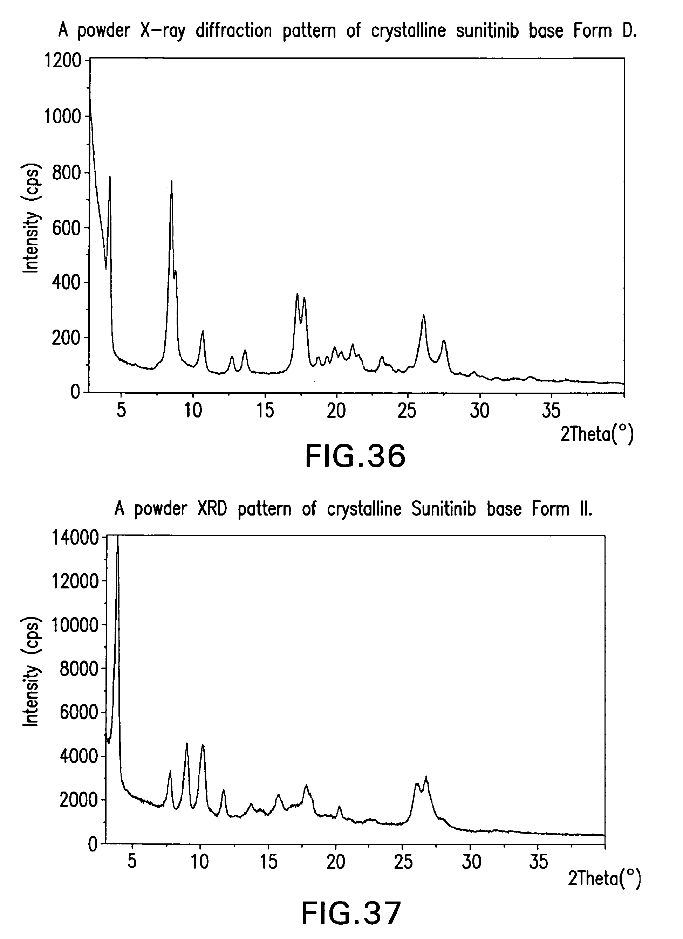

Figure 36 illustrate a powder X-ray diffraction pattern of crystalline sunitinib base Form D.
Figure 37 illustrate a powder XRD pattern of crystalline Sunitinib base Form II.

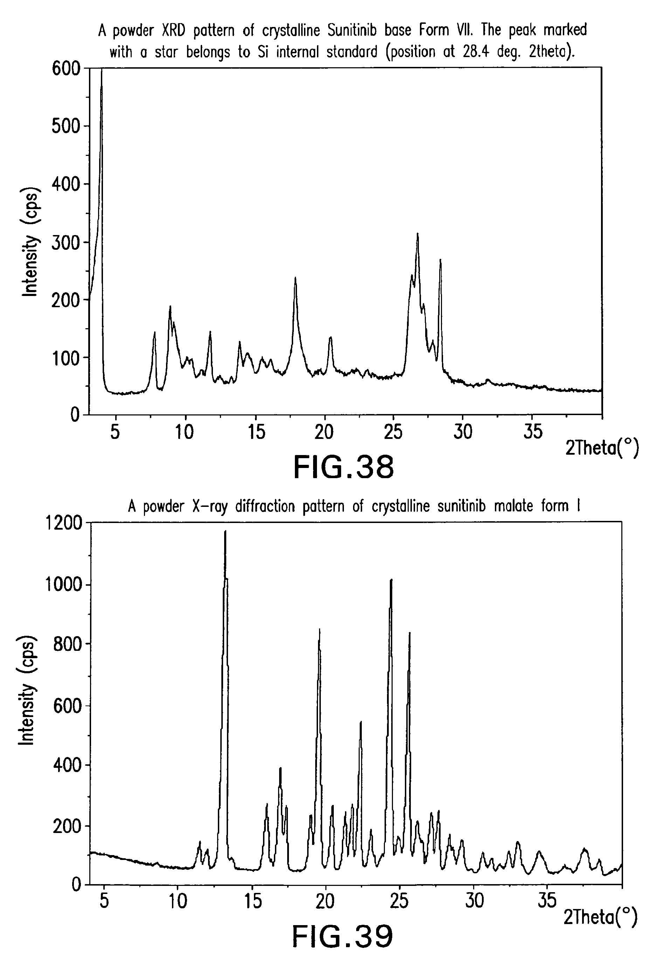

Figure 38 illustrate a powder XRD pattern of crystalline Sunitinib base Form VII.
Figure 39 shows a powder X-ray diffraction pattern of crystalline sunitinib malate form I.

### Detailed Description of the Invention

As used herein, the term "crystalline sunitinib base form VIII" refers to crystalline sunitinib base characterized by data selected from a group consisting of a PXRD pattern having any 5 peaks selected from a list consisting of: 3.8, 7.6, 8.5, 9.5, 10.4, 11.4, 16.5, 17.8, 20.6, and 27.0 deg ± 0.2 degrees 2-theta, a PXRD pattern having peaks at about 7.6 and 16.5 ± 0.2 degrees 2-theta and any 3 peaks at positions selected from the group consisting of 3.8, 8.5, 9.5, 11.4, 17.8, 20.6 and 27.0 deg ± 0.2 degrees 2-theta, a PXRD pattern as depicted in Figure 23 and combination thereof.

As used herein, the term "crystalline sunitinib base form X" refers to crystalline sunitinib base characterized by data selected from a group consisting of a PXRD pattern having any 5 peaks selected from a list consisting of: 3.9, 7.8, 9.1, 10.1, 11.6, 14.3, 15.9, 18.2, 20.4, 23.1, 23.9 and 27.0 deg ± 0.2 degrees 2-theta, a PXRD pattern having peaks at about 14.3 and 23.9 ± 0.2 degrees 2-theta and any 3 peaks at positions selected from the group consisting of 3.9, 7.8, 9.1, 10.1, 11.6, 23.1 and 27.0 deg ± 0.2 degrees 2-theta, a PXRD pattern as depicted in Figure 24, and combination thereof.

As used herein, the term "crystalline Sunitinib base form D" refers to crystalline Sunitinib base characterized by data selected from a group consisting of a PXRD pattern having any 5 peaks at positions selected from the group consisting of: 4.2, 8.5, 10.7, 12.7, 13.6, 17.2, 17.7, 21.1, 26.1 and 27.4 ± 0.2 degrees 2-theta, a PXRD pattern having peaks at about 3.9 and 4.2 ± 0.2 degrees 2-theta and 3 peaks selected from a list consisting of 8.5, 10.7, 13.6, 17.2, 17.7, 26.1 and 27.4 deg ± 0.2 degrees 2-theta, a PXRD pattern as depicted in Figure 36 and combination thereof.

As used herein, the term "crystalline Sunitinib base form II" refers to crystalline Sunitinib base characterized by crystalline Sunitinib base characterized by data selected from a group consisting of a PXRD pattern having any 5 peaks selected from the list consisting of: 3.8, 7.8, 9.0, 10.2, 11.8, 15.8, 17.9, 20.3, 26.1 and 26.8 deg± 0.2 degrees 2-theta, a PXRD pattern having peaks at about 9.0 and 26.1 ± 0.2 degrees 2-theta and any 3 peaks at positions selected from the group consisting of 3.8, 7.8, 10.2, 11.8, 15.8, 17.9, 20.3 and 26.8 deg ± 0.2 degrees 2-thetaa PXRD pattern as depicted in Figure 37 and combination thereof.

As used herein, the term "crystalline Sunitinib base form VII" refers to crystalline Sunitinib base characterized by data selected from a group consisting of a PXRD pattern having any 5 peaks selected from the list consisting of: 3.9, 7.8, 8.9, 9.2, 11.7, 13.9, 15.4, 16.0, 17.9, 20.4, 26.7 and 27.8 deg ± 0.2 degrees 2-theta, a PXRD pattern as depicted in Figure 38 and combination thereof.

As used herein, the term, "crystalline sunitinib malate form I" refers to crystalline form characterized by diffraction peaks at about 13.2 and 24.2 degrees two-theta, and more preferably, at about 13.2, 19.4, 24.2 and 25.5 degrees two-theta.

As used herein, the term "racemic" refers to a mixture that contains an equal amount of enantiomers.

The invention relates to Sunitinib hemi-L-malate, polymorphs thereof, polymorphs of racemic sunitinib malate, processes for preparation thereof, and pharmaceutical compositions thereof.

The invention also relates to compositions containing Sunitinib base and either L or racemic malic acid, possessing a superior solubility, for example in aqueous medium, than the solubility of sunitinib base.

The invention also relates to process for preparation thereof and pharmaceutical compositions thereof.

In one embodiment, the invention encompasses racemic sunitinib malate. Preferably, the racemic Sunitinib malate is solid, more preferably, crystalline.

In one embodiment, the invention encompasses a crystalline form of racemic sunitinib malate characterized by data selected from a group consisting of a PXRD pattern having any 5 peaks at positions selected from the group consisting of: 5.7, 8.0, 9.3, 12.5, 14.7, 15.4, 18.3, 21.5, 24.7 and 27.4 ± 0.2 degrees 2-theta, a PXRD pattern as depicted in Figure 1 and combination thereof. This crystalline form can be designated Form A

In a preferred embodiment, the present inventions encompasses crystalline racemic sunitinib malate designated form A characterized by a PXRD pattern having peaks at about 5.7 and 8.0 ± 0.2 degrees 2-theta and any 3 peaks at positions selected from the group consisting of 9.3, 12.5, 15.4, 18.3, 21.5, 24.7 and 27.4 ± 0.2 degrees 2-theta.

The crystalline racemic sunitinib malate Form A can be further characterized by data selected from the group consisting of: a PXRD pattern having peaks at about 5.7, 8.0, 12.5, 14.7 and 18.3 ± 0.2 degrees 2-theta; a PXRD pattern having peaks at about 5.7, 8.0, 9.3, 24.7 and 18.3 ± 0.2 degrees 2-theta; and a PXRD pattern having a single peak at about 27.4 ± 0.2 degrees 2-theta.

The crystalline racemic sunitinib malate Form A can be prepared by a process comprising reacting Sunitinib base and racemic malic acid in ethanol to obtain a suspension comprising the said crystalline form.

Preferably, the reaction of sunitinib base and racemic malic acid in ethanol provides a solution comprising racemic sunitinib malate, which is then precipitated to obtain the said suspension.

First, Sunitinib base, racemic malic acid and ethanol are combined providing a mixture. Preferably, the mixture is heated to obtain the said solution. Preferably, the heating is done to a temperature of about 50°C to about 78 °C. More preferably, the heating is done to a temperature of about 78°C.

Preferably, the precipitation of the said crystalline form A is done by cooling the said solution. Preferably, the cooling is done to a temperature of 15°C. Preferably, the cooling is conducted over night.

The process of preparing the said crystalline form A may further comprises recovering the crystalline racemic sunitinib malate from the suspension. The recovery may be done for example, by filtering the suspension, washing the precipitate and drying. Preferably, washing is done with ethanol. Preferably, drying is done at about room temperature.

In one embodiment, the invention encompasses a crystalline form of racemic sunitinib malate characterized by data selected from a group consisting of a PXRD pattern having any 5 peaks at positions selected from the group consisting of: 15.2, 16.1, 22.1, 23.5, 24.5, 27.2 and 27.5 ± 0.2 degrees 2-theta, a PXRD pattern as depicted in Figure 2 and combination thereof. This crystalline form can be designated Form B.

In a preferred embodiment, the present inventions encompasses crystalline racemic sunitinib malate designated form B characterized by a PXRD pattern having peaks at about 7.5 and 22.1 ± 0.2 degrees 2-theta and any 3 peaks at positions selected from the group consisting of 15.2, 16.1, 23.5, 24.5, 27.2 and 27.5 ± 0.2 degrees 2-theta.

The crystalline racemic sunitinib malate Form B can be further characterized by data selected from the group consisting of: a PXRD pattern having peaks at about 15.2, 16.1, 22.1, 27.2 and 27.5 ± 0.2 degrees 2-theta; and a PXRD pattern having a double peak at about 27.2 and 27.5 ± 0.2 degrees 2-theta.

The crystalline racemic sunitinib malate Form B can be prepared by a process comprising reacting sunitinib base and racemic malic acid in a mixture of dioxane and water to obtain a suspension comprising the said crystalline form.

Preferably, the reaction of sunitinib base and racemic malic acid in a mixture of dioxane and water provides a solution comprising racemic sunitinib malate, which is then precipitated to obtain the said suspension.

Preferably, the said mixture is provided by combining water and dioxane, wherein the water content is about 5 to about 20%. More preferably, the said mixture is provided by combining water and dioxane, wherein the water content is about 10%.

First, Sunitinib base and dioxane are combined providing a mixture. Preferably, the mixture is heated to obtain a first solution. Preferably, the heating is done to a temperature of about 101 °C. To this solution is then added an aqueous solution of racemic malic acid providing the said solution.

Preferably, the precipitation of the said crystalline form B is done by cooling the said solution. Preferably, the cooling is done to a temperature of about 12°C to about 30°C. more preferably, the cooling is done to a temperature of about 15°C. Preferably, the cooling is conducted over night. More preferably, cooling is conducted for about 30 minutes to about 24 hours. Most preferably, cooling is conducted for about 12 hours.

The process of preparing the said crystalline form B may further comprises recovering the crystalline racemic sunitinib malate from the suspension. The recovery may be done fore example, by filtering the suspension, washing the precipitate and drying. Preferably, washing is done with dioxane. Preferably, drying is done at about room temperature.

In one embodiment, the invention encompasses a composition containing sunitinib base and L-malic acid characterized by data selected from a group consisting of a PXRD pattern having any 5 peaks at positions selected from the group consisting of: 5.6, 9.5, 11.4, 13.0, 15.8, 16.8, 17.4, 18.3, 19.2, 20.9, 26.5 and 28.6 ± 0.2 degrees 2-theta, a PXRD pattern as depicted in Figure 3 and combination thereof. This composition can be designated as composition C.

In a preferred embodiment, the present inventions encompasses a composition containing Sunitinib base and L-malic acid designated composition C characterized by data selected from a group consisting of a PXRD pattern having peaks at about 13.0 and 14.8 ± 0.2 degrees 2-theta and any 3 peaks at positions selected from the group consisting of: 5.6, 9.5, 11.4, 15.8, 16.8, 18.3 and 26.5 ± 0.2 degrees 2-theta.

Composition C can be further characterized by data selected from the group consisting of: a PXRD pattern having peaks at about 5.6, 11.4, 18.3, 20.9 and 28.6 ± 0.2 degrees 2-theta; a PXRD pattern having peaks at about 5.6, 9.5, 14.8, 16.7 and 20.9 ± 0.2 degrees 2-theta and a PXRD pattern having peaks at about 9.5, 11.4, 14.8, 16.7 and 28.6 ± 0.2 degrees 2-theta.

Composition C can be prepared by a process comprising dissolving sunitinib malate in pyridine and precipitating the said solution to obtain a suspension comprising the said composition.

Preferably the said solution is provides by combining sunitinib malate and pyridine, and heating the combination. Preferably, the heating is done at a temperature of about 50°C to about 115°C. More preferably, heating is done to a temperature of about 100°C.

Preferably, the said solution is maintained at the said temperature for a period of about 15 minutes to about 2 hours. More preferably, the said solution is maintained at the said temperature for a period of about 30 minutes.

Preferably the precipitation occurs by cooling the said solution. Preferably, cooling is done to a temperature of about 30°C to about -40°C is reached. More preferably, cooling is done to a temperature of about 20°C to about -10°C is reached

Preferably, cooling is done for a period of about 30 minute to about 24 hours. More preferably, cooling is done for a period of about 12 hours.

In one embodiment, the invention encompasses Sunitinib hemi-L-malate, preferably, isolated Sunitinib hemi-L-malate, more preferably, solid Sunitinib hemi-L-malate, most preferably, crystalline Sunitinib hemi-L-malate.

In one embodiment, the invention encompasses a crystalline form of sunitinib hemi-L-malate characterized by data selected from a group consisting of a PXRD pattern having any 5 peaks at positions selected from the group consisting of: 3.7, 6.8, 10.3, 11.3, 11.9, 14.2, 15.1, 15.9, 25.9 and 26.6 ± 0.2 degrees 2-theta, a PXRD pattern as depicted in Figure 4, a PXRD pattern as depicted in Figure 4a and combination thereof. This crystalline form can be designated Form E.

In a preferred embodiment, the present inventions encompasses crystalline sunitinib hemi-L-malate designated form E characterized by a PXRD pattern having peaks at about 6.8 and 11.3 ± 0.2 degrees 2-theta and any 3 peaks at positions selected from the group consisting of: 3.7, 11.9, 15.1, 25.9 and 26.6 ± 0.2 degrees 2-theta.

Crystalline sunitinib hemi-L-malate can be further characterized by data selected from the group consisting of: a PXRD pattern having peaks at about 3.7, 6.8, 11.3, 11.9 and 25.9 ± 0.2 degrees 2-theta.

The crystalline sunitinib hemi-L-malate Form E can be prepared by a process comprising lyophilizing an aqueous solution of sunitinib hemi-L-malate. In the lyophilization process, the said aqueous solution is subjected to a pressure of less than about one atmosphere, to remove solvent. Preferably, the solvent is removed at a pressure of about 0.05 to about 50 mBar. More preferably, the solvent is removed at a pressure of about 0.5 to about 5 mBar. Most preferably, the solvent is removed at a pressure of about 1 mBar. Preferably, the solvent is removed at a temperature of about 20°C.

Typically, the aqueous solution is provided by combining sunitinib base, water and L-malic acid, and heating the combination. Preferably, L-malic acid is combined in a ratio of about 0.5 mole equivalent per mole equivalent of sunitinib base.

In one embodiment, the invention encompasses a composition containing sunitinib base and L-malic acid characterized by data selected from a group consisting of a PXRD pattern having any 5 peaks at positions selected from the group consisting of: 6.0, 7.7, 9.1, 10.1, 12.0, 14.5, 23.4 and 27.1 ± 0.2 degrees 2-theta, a PXRD pattern as depicted in Figure 5, a PXRD pattern as depicted in Figure 5a and combination thereof. This composition can be designated as composition F.

In a preferred embodiment, the present inventions encompasses a composition containing Sunitinib base and L-malic acid designated composition F characterized by a PXRD pattern having peaks at about 12.0 and 23.4 ± 0.2 degrees 2-theta and any 3 peaks at positions selected from the group consisting of: 6.0, 7.7, 9.1, 10.1, 14.5 and 27.1 ± 0.2 degrees 2-theta.

The above composition F can be further characterized by data selected from the group consisting of: a PXRD pattern having peaks at about 7.7, 10.1, 12.0, 23.4 and 27.1 ± 0.2 degrees 2-theta; and a PXRD pattern having peaks at about 6.0, 10.1, 12.0, 23.4 and 27.1 ± 0.2 degrees 2-theta.

Composition F can be prepared by a process comprising providing slurry comprising sunitinib base, L-malic acid and methyl tetr-butyl ether ("MTBE") at about room temperature.

Preferably, the slurry is prepared by combining sunitinib base, L-malic acid and MTBE at about room temperature, wherein Sunitinib base contains about 15% of water.

. Preferably, the slurry is maintained in an ultrasound bath, prior to recovery the said composition F.

The process may further comprise recovering the composition from the slurry, for example by filtering and drying.

In one embodiment, the invention encompasses a composition containing sunitinib base and L-malic acid characterized by data selected from a group consisting of a PXRD pattern having any 5 peaks at positions selected from the group consisting of: 6.2, 7.7, 9.3, 12.4, 14.5, 23.2 and 27.4 ± 0.2 degrees 2-theta, a PXRD pattern as depicted in Figure 6, a PXRD pattern as depicted in Figure 6a and combination thereof. This composition can be designated as composition G.

In a preferred embodiment, the present inventions encompasses a composition containing Sunitinib base and L-malic acid designated composition G characterized by a PXRD pattern having peaks at about 9.3 and 12.4 ± 0.2 degrees 2-theta and any 3 peaks at positions selected from the group consisting of: 6.2, 7.7, 14.5, 23.2 and 27.4 ± 0.2 degrees 2-theta.

The above composition G can be further characterized by data selected from the group consisting of: a PXRD pattern having peaks at about 6.2, 9.3, 12.4 and 23.2 ± 0.2 degrees 2-theta; and a PXRD pattern having peaks at about 7.7, 9.3, 12.4 and 27.4 ± 0.2 degrees 2-theta.

Composition G can be prepared by a process comprising dissolving Sunitinib malate in water and lyophilizing the said solution to obtain composition G.

In the lyophilization process, the said solution is first frozen and then is subjected to a pressure of less than about one atmosphere, to remove solvent. Preferably, the solvent is removed at a pressure of about 0.05 to about 50 mBar. More preferably, the solvent is removed at a pressure of about 0.5 to about 5 mBar. Most preferably, the solvent is removed at a pressure of about 1 mBar. Preferably, the solvent is removed at a temperature of about 25 °C.

Typically, the said solution is provided by combining sunitinib base, water and L-malic acid and heating the said combination. Preferably, heating is done to a temperature of about 100°C. Preferably, heating is preformed for a period of about 10 minutes.

In one embodiment, the invention encompasses a composition containing Sunitinib base and L-malic acid characterized by data selected from a group consisting of a PXRD pattern having any 5 peaks at positions selected from the group consisting of: 6.0, 7.8, 9.0, 12.0, 14.8, 18.0, 22.5 and 27.1 ± 0.2 degrees 2-theta, a PXRD pattern as depicted in Figure 7, a PXRD pattern as depicted in Figure 7a and combination thereof. This composition can be designated as composition H.

In a preferred embodiment, the present inventions encompasses a composition containing Sunitinib base and L-malic acid designated composition H characterized by a PXRD pattern having peaks at about 9.0 and 12.0 ± 0.2 degrees 2-theta and any 3 peaks at positions selected from the group consisting of: 6.0, 7.8, 14.8, 18.0, 22.5 and 27.1 ± 0.2 degrees 2-theta.

The above composition H can be further characterized by data selected from the group consisting of: a PXRD pattern having peaks at about 6.0, 9.0, 12.0, 18.0 and 27.1 ± 0.2 degrees 2-theta; and a PXRD pattern having peaks at about 9.0, 12.0, 14.8, 18.0 and 27.1 ± 0.2 degrees 2-theta.

Composition H can be prepared by a process comprising providing a slurry comprising sunitinib base, L-malic acid and MTBE at about reflux temperature. Preferably, the slurry is prepared by combining sunitinib base, L-malic acid and MTBE, and heating the combination to about reflux temperature. Preferably, heating is done to a temperature of about 60°C to about 100°C. More preferably, heating is done to a temperature of about 100°C.

The process may further comprise recovering the composition from the slurry, for example by filtering and drying.

In one embodiment, the invention encompasses a composition containing Sunitinib base and L-malic acid characterized by data selected from a group consisting of a PXRD pattern having any 5 peaks at positions selected from the group consisting of: 6.0, 7.7, 9.2, 12.2, 14.5, 22.9 and 27.3 ± 0.2 degrees 2-theta, a PXRD pattern as depicted in Figure 8, a PXRD pattern as depicted in Figure 8a and combination thereof. This composition can be designated as composition I.

In a preferred embodiment, the present inventions encompasses a composition containing Sunitinib base and L-malic acid designated composition I characterized by a PXRD pattern having peaks at about 6.0 and 12.2 ± 0.2 degrees 2-theta and any 3 peaks at positions selected from the group consisting of: 7.7, 9.2, 14.5, 22.9 and 27.3 ± 0.2 degrees 2-theta..

The above composition I can be further characterized by data selected from the group consisting of: a PXRD pattern having peaks at about 6.0, 9.2, 12.2, 14.5 and 22.9 ± 0.2 degrees 2-theta; and a PXRD pattern having peaks at about 7.7, 12.2, 14.5, 22.9 and 27.4 ± 0.2 degrees 2-theta.

Composition I can be prepared by a process comprising combining sunitinib base and L-malic acid in water to obtain a solution, and lyophilizing the solution; wherein the ratio of L-malic acid to water is about 37:1 w/v, respectively.

Preferably, the ratio of Sunitinib base to L-malic acid is 1:1.1 respectively.

In the lyophilization process, the said aqueous solution is subjected to a pressure of less than about one atmosphere, to remove solvent. Preferably, the solvent is removed at a pressure of about 0.05 to about 50 mBar. More preferably, the solvent is removed at a pressure of about 0.5 to about 5 mBar. Most preferably, the solvent is removed at a pressure of about 1 mBar. Preferably, the solvent is removed at a temperature of about 25 °C. Typically, t h e aqueous solution is provided by combining sunitinib base, water and L-malic acid, and heating the combination. Preferably, the heating is done to a temperature of about 100 °C.

In one embodiment, the invention encompasses a composition containing Sunitinib base and L-malic acid characterized by data selected from a group consisting of a PXRD pattern having any 5 peaks at positions selected from the group consisting of: 5.8, 7.7, 8.7, 11.7, 13.3, 14.5, 22.6 and 27.2 ± 0.2 degrees 2-theta, a PXRD pattern as depicted in Figure 9, a PXRD pattern as depicted in Figure 9a and combination thereof. This composition can be designated as composition J.

In a preferred embodiment, the present inventions encompasses a composition containing Sunitinib base and L-malic acid designated composition J characterized by a PXRD pattern having peaks at about 11.7 and 22.6 ± 0.2 degrees 2-theta and any 3 peaks at positions selected from the group consisting of: 5.8, 7.7, 8.7, 13.3, 14.5 and 27.2 ± 0.2 degrees 2-theta

The above composition J can be further characterized by data selected from the group consisting of: a PXRD pattern having peaks at about 7.7, 11.7, 13.3, 22.6 and 27.2 ± 0.2 degrees 2-theta; and a PXRD pattern having peaks at about 5.8, 8.7, 11.7, 13.3 and 22.6 ± 0.2 degrees 2-theta.

Composition J can be prepared by a process comprising combining sunitinib base and L-malic acid in water to obtain a solution, and lyophilizing the solution; wherein the ratio of: water is about 50:1 w/v, respectively.

Preferably, the ratio of Sunitinib base to L-malic acid is 1:1.5, respectively.

In the lyophilization process, the said aqueous solution is subjected to a pressure of less than about one atmosphere, to remove solvent. Preferably, the solvent is removed at a pressure of about 0.05 to about 50 mBar. More preferably, the solvent is removed at a pressure of about 0.5 to about 5 mBar. Most preferably, the solvent is removed at a pressure of about 1 mBar. Preferably, the solvent is removed at a temperature of about 25 °C. Typically, the aqueous solution is provided by combining sunitinib base, water and L-malic acid, and heating the combination. Preferably, heating is done to a temperature of about 60°C to about 100°C. More preferably, heating is done to a temperature of about 100°C.

In one embodiment, the invention encompasses a composition containing Sunitinib base and L-malic acid characterized by data selected from a group consisting of a PXRD pattern having peaks at positions selected from the group consisting of: 11.4, 14.4, 23.4, 24.1 and 27.0 ± 0.2 degrees 2-theta, a PXRD pattern as depicted in Figure 10, a PXRD pattern as depicted in Figure 10a and combination thereof. This composition can be designated as composition K.

Composition K can be further characterized by data selected from the group consisting of: a PXRD pattern having peaks at about 11.4, 14.4, 23.4 and 24.1 ± 0.2 degrees 2-theta; and a PXRD pattern having double peak at about 23.4 and 24.1 ± 0.2 degrees 2-theta.

Composition K can be prepared by a process comprising combining sunitinib base and L-malic acid in water to obtain a solution, and lyophilizing the solution; wherein the ratio of L-malic acid to water is about 67:1 w/v, respectively.

Preferably, the ratio of Sunitinib base to L-malic acid is 1:2 respectively.

In the lyophilization process, the said aqueous solution is subjected to a pressure of less than about one atmosphere, to remove solvent. Preferably, the solvent is removed at a pressure of about 0.05 to about 50 mBar. More preferably, the solvent is removed at a pressure of about 0.5 to about 5 mBar. Most preferably, the solvent is removed at a pressure of about 1 mBar.

Preferably, the solvent is removed at a temperature of about 20 °C. Typically, the aqueous solution is provided by combining sunitinib base, water and L-malic acid, and heating the combination. Preferably, heating is done to a temperature of about 60°C to about 100°C. More preferably, heating is done to a temperature of about 100°C.

In one embodiment, the invention encompasses a composition containing Sunitinib base and L-malic acid characterized by data selected from a group consisting of a PXRD pattern having any 5 peaks at positions selected from the group consisting of: 6.2, 7.6, 9.3, 12.4, 14.6, 22.9 and 27.4 ± 0.2 degrees 2-theta, a PXRD pattern as depicted in Figure 11, a PXRD pattern as depicted in Figure 11a and combination thereof. This composition can be designated as composition L.

In a preferred embodiment, the present inventions encompasses a composition containing Sunitinib base and L-malic acid designated composition L characterized by a PXRD pattern having peaks at about 14.6 and 22.9 ± 0.2 degrees 2-theta and any 3 peaks at positions selected from the group consisting of: 6.2, 7.6, 9.3, 12.4 and 27.4 ± 0.2 degrees 2-theta.

The above composition L can be further characterized by data selected from the group consisting of: a PXRD pattern having peaks at about 6.2, 9.3, 14.6, 22.9 and 27.4± 0.2 degrees 2-theta; and a PXRD pattern having peaks at about 7.6, 9.3, 12.4, 14.6 and 22.9 ± 0.2 degrees 2-theta.

Composition L can be prepared by a process comprising reacting sunitinib base and L-malic acid in water to obtain a solution, and lyophilizing the solution; wherein the ratio of L-malic acid to water is about 34:1 w/v, respectively.

Preferably, the ratio of Sunitinib base to L-malic acid is 1:1 respectively.

In the lyophilization process, the said aqueous solution is subjected to a pressure of less than about one atmosphere, to remove solvent. Preferably, the solvent is removed at a pressure of about 0.05 to about 50 mBar. More preferably, the solvent is removed at a pressure of about 0.5 to about 5 mBar. Most preferably, the solvent is removed at a pressure of about 1 mBar.

Preferably, the solvent is removed at a temperature of about 25 °C.

Typically, the aqueous solution is provided by combining sunitinib base, water and L-malic acid, and heating the combination. Preferably, the heating is done to a temperature of about 100 °C.

In one embodiment, the invention encompasses a composition containing Sunitinib base and L-malic acid characterized by data selected from a group consisting of a PXRD pattern having any 5 peaks at positions selected from the group consisting of: 6.0, 7.7, 9.2, 12.3, 14.5, 23.0 and 27.3 ± 0.2 degrees 2-theta, a PXRD pattern as depicted in Figure 12, a PXRD pattern as depicted in Figure 12a and combination thereof. This composition can be designated as composition M.

In a preferred embodiment, the present inventions encompasses a composition containing Sunitinib base and L-malic acid designated composition M characterized by a PXRD pattern having peaks at about 9.2 and 12.3 ± 0.2 degrees 2-theta and any 3 peaks at positions selected from the group consisting of: 6.0, 7.7, 14.5, 23.0 and 27.3 ± 0.2 degrees 2-theta

The above composition M can be further characterized by data selected from the group consisting of: a PXRD pattern having peaks at about 6.0, 9.2, 14.5, 23.0 and 27.3 ± 0.2 degrees 2-theta; and a PXRD pattern having peaks at about 7.7, 9.2, 12.3, 14.5 and 23.0 ± 0.2 degrees 2-theta.

Composition M can be prepared by a process comprising heating Sunitinib malate to a temperature of about 50°C to 100°C. Preferably, heating is done to a temperature of about 80°C. Preferably, heating is done for a period of about 30 minutes to about 4 hours. More preferably, heating is done for a period of about 2 hours. Preferably, the heating is done at a pressure of about 0.05 to about 50 mBar. More preferably, the solvent is removed at a pressure of about 0.5 to about 5 mBar. Most preferably, the solvent is removed at a pressure of about 1 mBar.

In one embodiment, the invention encompasses a composition containing Sunitinib base and L-malic acid characterized by data selected from a group consisting of a PXRD pattern having any 5 peaks at positions selected from the group consisting of: 6.0, 7.6, 9.2, 12.1, 14.5, 24.2 and 27.2 ± 0.2 degrees 2-theta, a PXRD pattern as depicted in Figure 13, a PXRD pattern as depicted in Figure 13a and combination thereof. This composition can be designated as composition N.

In a preferred embodiment, the present inventions encompasses a composition containing Sunitinib base and L-malic acid designated composition N characterized by a PXRD pattern having peaks at about 7.6 and 27.2 ± 0.2 degrees 2-theta and any 3 peaks at positions selected from the group consisting of: 6.0, 9.2, 12.1, 14.5 and 24.2 ± 0.2 degrees 2-theta.

The above composition N can be further characterized by data selected from the group consisting of: a PXRD pattern having peaks at about 6.0, 9.2, 12.1, 24.2 and 27.2 ± 0.2 degrees 2-theta; and a PXRD pattern having peaks at about 6.0, 7.6, 9.2, 12.1 and 27.2 ± 0.2 degrees 2-theta.

Composition N can be prepared by a process comprising dissolving sunitinib malate in a mixture of dioxane and water, and lyophilizing the solution. In the lyophilization process, the said solution is subjected to a pressure of less than about one atmosphere, to remove solvent.

Typically, the solution is provided by combining sunitinib malate and a mixture of dioxane and water. Preferably, the mixture contains about 5 to about 50% of water (v/v). More preferably, the mixture contains about 5 to about 10% of water (v/v). Most preferably, the mixture contains about 5% of water (v/v).

In one embodiment, the invention encompasses a composition containing Sunitinib base and L-malic acid characterized by data selected from a group consisting of a PXRD pattern having any 5 peaks at positions selected from the group consisting of: 6.0, 7.4, 8.9, 11.9, 23.4 and 27.7 ± 0.2 degrees 2-theta, a PXRD pattern as depicted in Figure 14, a PXRD pattern as depicted in Figure 14a and combination thereof. This composition can be designated as composition O.

In a preferred embodiment, the present inventions encompasses a composition containing Sunitinib base and L-malic acid designated composition O characterized by a PXRD pattern having peaks at about 6.0 and 11.9 ± 0.2 degrees 2-theta and any 3 peaks at positions selected from the group consisting of: 7.4, 8.9, 23.4 and 27.7 ± 0.2 degrees 2-theta.

The above composition 0 can be further characterized by a PXRD pattern having four peaks at about 6.0, 7.4, 8.9 and 11.9 ± 0.2 degrees 2-theta.

The composition O can be prepared by a process comprising combining sunitinib base, L-malic acid, and a mixture of water, and tetrahydrofuran and evaporating the solvent.

Typically, the process for preparing the said composition O comprises combining sunitinib base, L-maiic acid in a mixture of water and tetrahydrofuran, and heating the said combination to obtain a solution. Preferably, heating is done to a temperature of about 40°C to about 66°C. More preferably, heating is done to a temperature of about 66°C.

Preferably, the mole ratio of L-malic acid and water is about 1:1.

The evaporation of the solvent from the said solution is done at a temperature of about 40°C to about 10°C, providing the said composition O. More preferably, evaporation is done at a temperature of about 20 °C.

In one embodiment, the invention encompasses a composition containing Sunitinib base and L-malic acid characterized by data selected from a group consisting of a PXRD pattern having any 5 peaks at positions selected from the group consisting of: 6.1, 7.9, 9.2, 12.1, 15.2, 22.9 and 27.7 ± 0.2 degrees 2-theta, a PXRD pattern as depicted in Figure 15, a PXRD pattern as depicted in Figure 15a and combination thereof. This composition can be designated as composition P.

In a preferred embodiment, the present inventions encompasses a composition containing Sunitinib base and L-malic acid designated composition P characterized by a PXRD pattern having peaks at about 9.2 and 15.2 ± 0.2 degrees 2-theta and any 3 peaks at positions selected from the group consisting of: 6.1, 7.9, 12.1, 22.9 and 27.7 ± 0.2 degrees 2-theta.

The above composition P can be further characterized by a PXRD pattern having four peaks at about 9.2, 12.1, 15.2, 22.9 and 27.7 ± 0.2 degrees 2-theta.

Composition P can be prepared by a process comprising combining sunitinib base, L-malic acid and a mixture of water and dioxolane, and evaporating the solvent.

Typically, the process for preparing the said composition P comprises combining sunitinib base, L-malic acid in water and dioxolane, and heating the said combination to obtain a solution.

Preferably, heating is done to a temperature of about 40°C to about 76°C. More preferably, heating is done to a temperature of about 76°C.

Preferably, the mole ratio of L-malic acid and water is about 1:1.

The evaporation of the solvent from the said solution is done at a temperature of about 40°C to about 10°C, providing the said composition P. More preferably, evaporation is done at a temperature of about 20 °C.

In one embodiment, the invention encompasses a composition containing Sunitinib base and L-malic acid characterized by data selected from a group consisting of a PXRD pattern having any 5 peaks at positions selected from the group consisting of: 5.9, 8.9, 11.8, 20.6, 22.6 and 27.3 ± 0.2 degrees 2-theta, a PXRD pattern as depicted in Figure 16, a PXRD pattern as depicted in Figure 16a and combination thereof. This composition can be designated as composition Q.

In a preferred embodiment, the present inventions encompasses a composition containing Sunitinib base and L-malic acid characterized by a PXRD pattern having peaks at about 8.9 and 11.8 ± 0.2 degrees 2-theta and any 3 peaks at positions selected from the group consisting of: 5.9, 20.6, 22.6 and 27.3 ± 0.2 degrees 2-theta.

The above composition Q can be further characterized by a PXRD pattern having four peaks at about 5.9, 8.9, 11.8 and 27.3 ± 0.2 degrees 2-theta.

Composition Q can be prepared by a process comprising evaporating a solution comprising sunitinib base, L-malic acid and a mixture of water and dioxane.

Preferably, the solution is provided by combining sunitinib base, L-malic acid and a mixture of water and dioxane, and heating the said combination to obtain a solution.

Preferably, the mole ratio of L-malic acid and water is about 1:1. Preferably, the heating is done to a temperature of about 40°C to about 100°C. More preferably, heating is done to a temperature of about 100°C.

Preferably, evaporation of the solvent is done at a temperature of about 40°C to about 10°C, providing the said composition Q. More preferably, evaporation is done at a temperature of about 20 °C.

In one embodiment, the invention encompasses a composition containing Sunitinib base and L-malic acid characterized by data selected from a group consisting of a PXRD pattern any 5 peaks at positions selected from the group consisting of: 3.4, 5.6, 9.6, 10.3, 17.8, 18.4 and 26.0 ± 0.2 degrees 2-theta, a PXRD pattern as depicted in Figure 17 and combination thereof. This composition can be designated as composition R.

In a preferred embodiment, the present inventions encompasses a composition containing Sunitinib base and L-malic acid characterized designated composition R by a PXRD pattern having peaks at about 3.4 and 18.4 ± 0.2 degrees 2-theta and any 3 peaks at positions selected from the group consisting of: 5.6, 9.6, 10.3, 17.8 and 26.0 ± 0.2 degrees 2-theta.

The above composition R can be further characterized by data selected from a group consisting of: a powder XRD pattern with peaks at about 3.4, 5.6, 9.6, 10.3 and 17.8 ± 0.2 degrees 2-theta; and a powder XRD pattern with peaks at about 5.6, 10.3, 17.8, 18.4 and 26.0 ± 0.2 degrees 2-theta.

The composition R can be prepared by a process comprising providing solution comprising Sunitinib base, L-malic acid and dimethylsulfoxide ("DMSO"), and lyophilizing the solution. In the lyophilization process, the said solution is subjected to a pressure of less than about one atmosphere, to remove solvent.

Typically, the solution is provided by combining sunitinib base, DMSO and L-malic acid.

In one embodiment, the invention encompasses a crystalline form of sunitinib hemi-L-malate characterized by data selected from a group consisting of: a PXRD pattern any 5 peaks at positions selected from the group consisting of: 5.8, 9.6, 13.9, 15.9, 22.7, 26.6 and 28.7 ± 0.2 degrees 2-theta, a solid-state ¹³C NMR spectrum with signals at about 169.0, 136.0 and 119.5 ± 0.2 ppm, a solid-state ¹³C NMR spectrum having chemical shifts differences between the signal exhibiting the lowest chemical shift and another in the chemical shift range of 100 to 180 ppm of about 66.3, 33.3, 16.8 ± 0.1 ppm, a PXRD pattern as depicted in Figure 18; a ¹³C NMR spectrum depicted in Figure 19, and a solid-state ¹³C NMR spectrum depicted in Figure 20 and combination thereof. This form can be designated as form U.

Typically, the signal exhibiting the lowest chemical shift in the chemical shift area of 100 to 180 ppm is at about 102.7 ± 1 ppm. This form can be designated as form U.

In a preferred embodiment, the present inventions encompasses a crystalline form of Sunitinib hemi-L-malate designated form U characterized by a PXRD pattern having peaks at about 5.8 and 22.7 ± 0.2 degrees 2-theta and any 3 peaks at positions selected from the group consisting of: 9.6, 13.9, 15.9, 26.6 and 28.7 ± 0.2 degrees 2-theta

The above sunitinib hemi-L-malate form U can be further characterized by data selected from a group consisting of: a powder XRD pattern with peaks at about 5.8, 13.9, 22.7, 26.6 and 28.7 ± 0.2 degrees 2-theta; a powder XRD pattern with peaks at about 5.8, 9.6, 22.7, 26.6 and 28.7 ± 0.2 degrees 2-theta; a thermogram depicted in Figure 21 and having DSC peak at about 218.8 ± 2 °C; a solid-state ¹³C NMR spectrum having signals at about 156.6 and 117.5 ± 0.2 ppm; and a solid-state ¹³C NMR spectrum having chemical shifts differences between the signal exhibiting the lowest chemical shift and another in the chemical shift range of 100 to 180 ppm of about 53.9 and 14.8 ± 0.1 ppm.

The above sunitinib hemi-L-malate form U is anhydrous. As used herein, the term "anhydrous" in reference to form U of sunitinib hemi-L-malate, refers to form U of sunitinib hemi-L-malate that contains no more than about 0.5% by weight of water or of any organic solvent, as measured by TGA.

The crystalline sunitinib hemi-L-malate Form U can be prepared by a process comprising crystallizing sunitinib hemi-L-malate from a mixture of pyridine and methanol.

Preferably, the crystallization comprises providing a solution of sunitinib hemi-L-malate in a mixture of pyridine and methanol, and precipitating the said crystalline form to obtain a suspension.

Preferably, the said solution is provided by dissolving sunitinib base in pyridine to obtain a fist solution, and reacting the first solution with a second solution of L-malic acid in methanol. Preferably, the mole ratio between the reacted sunitinib base and L-malic acid is of about 1:1, however not all L-malic acid reacts. Thus, Sunitinib hemi-L-malate is obtained..

Preferably, prior to reacting the two solutions, the first solution is frozen to a temperature of about 100-230K. More preferably, the first solution is frozen to a temperature of about 150 K.

Preferably, the reaction of the two solutions provides a mixture comprising of sunitinib hemi-L-malate. Preferably, the reaction mixture is heated to a temperature of about --10°C to about -40°C. More preferably, the reaction mixture is heated to a temperature of about -30°C.

Preferably, the said mixture is maintained at the above temperature for a period of about 5 days, to allow precipitation of the said crystalline form.

The process of preparing the said crystalline form U may further comprises recovering the said crystalline Sunitinib hemi-L-malate from the said suspension. The recovery may be done for example, by filtering, washing and drying. Preferably, the washing is done with t-butyl methyl ester. Preferably, the drying is done by air.

In one embodiment, the invention encompasses a composition containing Sunitinib base and L-malic acid, characterized by data selected from a group consisting of PXRD pattern having any 5 peaks at positions selected from the group consisting of: 8.5, 9.3, 16.5, 17.8, 20.9 and 29.7 ± 0.2 degrees 2-theta, a PXRD pattern as depicted in Figure 25 and combination thereof. This composition can be designated as composition V-A.

In a preferred embodiment, the invention encompasses a composition containing Sunitinib base and L-malic acid designated composition V-A, characterized by a PXRD pattern having peaks at about: 8.5, and 21.0 deg ± 0.2 degrees 2-theta, and any 3 peaks at positions selected from the group consisting of: 3.8, 9.3, 16.5, 17.8 and 29.7 ± 0.2 degrees 2-theta.

Composition V-A can be further characterized by data selected from the group consisting of: a PXRD pattern having peaks at about: 8.5, 9.3, 16.5, 17.8, and 20.9± 0.2 degrees 2-theta; a PXRD pattern having peaks at about: 9.3, 16.5, 17.8, 20.9, and 29.7± 0.2 degrees 2-theta; a DSC thermogram having two peaks at around: 173 °C with onset temperature at around 159 °C and around 196 °C with onset temperature at around 163 °C., and a DSC thermogram as depicted in Figure 28.

The above composition V-A has solubility in water which is greater than 30 mg/ml, in comparison to the solubility of sunitinib base form VIII which is less than 1 mg/ml. Hence, such a composition would be more suitable for preparing a pharmaceutical composition.

Composition V-A containing Sunitinib base and L-malic acid can be prepared by a process comprising combining the crystalline Sunitinib base characterized by PXRD pattern having any 5 peaks selected from a list consisting of: 3.8, 7.6, 8.5, 9.5, 10.4, 11.4, 16.5, 17.8, 20.6, and 27.0 deg ± 0.2 degrees 2-theta, designated form VIII, and crystalline L-malic acid.

Typically, the process for preparing the said composition V-A comprises mixing a powder of crystalline sunitinib base form VIII and a powder of crystalline L-malic acid. Preferably, both powders are grounded separately, prior to mixing them. Preferably, when combined, a homogenizing powdery mixture comprising of the said composition is formed. Preferably, grounding is done in mortar by means of pestle. Preferably, homogenizing is done by shaking.

In one embodiment, the present invention encompasses a composition containing Sunitinib base and L-malic acid characterized by data selected from a group consisting of a PXRD pattern having any 5 peaks at positions selected from the group consisting of: 3.8, 14.3, 14.9, 17.8 and 27.0 ± 0.2 degrees 2-theta, a PXRD pattern as depicted in Figure 26 and combination thereof. This composition can be designated as composition V-B.

In a preferred embodiment, the present invention encompasses a composition containing Sunitinib base and L-malic acid, characterized by PXRD pattern having peaks at about 3.8 and 14.3 ± 0.2 degrees 2-theta and any 3 peaks selected from a list consisting of: 7.6, 8.5, 14.9, 17.8 and 27.0 ± 0.2 degrees 2-theta.

Composition V-B can be further characterized by data selected from the group consisting of: a PXRD pattern having peaks at about: 3.8, 14.3, 14.9, and 17.8± 0.2 degrees 2-theta; a PXRD pattern having peaks at about: 3.8, 14.3, 14.9, and 27.0± 0.2 degrees 2-theta; and a DSC thermogram having two peaks at around: 173 °C with onset temperature at around 159 °C and at around 194°C with onset temperature at around 164 °C, a DSC thermogram as depicted in Figure 29.

Composition V-B containing Sunitinib base and L-malic acid can be prepared by a process comprising heating the above composition V-A containing.

Preferably, the heating is to a temperature of about 70°C to about 90°C. More preferably, the heating is to a temperature of about 80°C. Preferably, the heating is done for a period of about 3 to about 5 hours. More preferably, heating is done for a period of about 4 hours.

In one embodiment, the present invention encompasses a composition containing Sunitinib base and L-malic acid characterized by data selected from a group consisting of a PXRD pattern having any 5 peaks at positions selected from the group consisting of: 5.5, 8.4, 11.1, 19.5 and 26.9 ± 0.2 degrees 2-theta, a PXRD pattern as depicted in Figure 27 and combination thereof. This composition can be designated as composition V-C.

In a preferred embodiment, the present invention encompasses composition containing Sunitinib base and L-malic acid, characterized by PXRD pattern having peaks at about 5.5 and 11.1 ± 0.2 degrees 2-theta and any 3 peaks selected from a list consisting of: 8.4, 19.5 and 26.9 ± 0.2 degrees 2-theta.

Composition V-C can be further characterized by data selected from the group consisting of: a PXRD pattern having peaks at about: 5.5, 8.4, 11.1, and 19.5± 0.2 degrees 2-theta; a PXRD pattern having peaks at about: 5.5, 11.1, 19.5, and 26.9± 0.2 degrees 2-theta.

Composition V-C containing Sunitinib base and L-malic acid can be prepared by a process comprising slurring composition V-A containing Sunitinib base and L-malic acid in water.

Preferably, the slurry is maintained at a temperature of about 20°C to about 30°C. more preferably, the slurry is maintained at a temperature of about 25°C.

Preferably, the slurry is maintained for a period of about 3 days.

In one embodiment, the invention encompasses a composition containing Sunitinib base and L-malic acid, characterized by data selected from a group consisting of: a PXRD pattern having any 5 at positions peaks selected from the group consisting of 8.5, 11.3, 23.2, 24.0 and 26.9 ± 0.2 degrees 2-theta and a solid-state ¹³C NMR spectrum with peaks at about 170.5, 157.2 and 115.6 ± 0.2 ppm, a solid-state ¹³C NMR spectrum having chemical shifts differences between the signal exhibiting the lowest chemical shift and another in the chemical shift range of 100 to 180 ppm of about 65.9, 52.6 and 11.0 ± 0.1 ppm, a PXRD pattern as depicted in Figure 30, a solid-state ¹³C NMR spectrum depicted in Figure 31, a solid-state ¹³C NMR spectrum depicted in Figure 32 and combination thereof. This composition can be designated as composition V-S.

In a preferred embodiment, the invention encompasses a composition containing Sunitinib base and L-malic acid, characterized by a PXRD pattern having peaks at about 8.5 and 11.3 ± 0.2 degrees 2-theta and any 3 peaks selected from a group consisting of 14.2, 23.2, 24.0 and 26.9 ± 0.2 degrees 2-theta.

Typically, the signal exhibiting the lowest chemical shift in the chemical shift area of 100 to 180 ppm is at about 104.6 ± 1 ppm.

Composition V-S of sunitinib and L-malic acid can be further characterized by data selected from a group consisting of: a solid-state ¹³C NMR spectrum having signals at about 139.4 and 126.0 ± 0.2 ppm; a solid-state ¹³C NMR spectrum having chemical shifts differences between the signal exhibiting the lowest chemical shift and another in the chemical shift range of 100 to 180 ppm of about 34.8 and 21.4 ± 0.1 ppm.

Composition V-S can be prepared by a process comprising lyophilizing an aqueous solution comprising Sunitinib base form X, L-malic acid and water, wherein the ratio of L-malic acid to water is about 29.7:1 w/v, respectively.

Preferably, the ratio of Sunitinib base to L-malic acid is 1:2 respectively.

Lyophilization is also known as freeze-drying.

In the lyophilization process, the said aqueous solution is subjected to a pressure of less than about one atmosphere, to remove the solvent.

Preferably, the aqueous solution is provided by a process comprising combining Sunitinib base form X, L-malic acid and water and heating the said combination. Preferably, the mole ratio between Sunitinib base form X and L-malic acid is of about 1:2.

Preferably, freezing the solution is done gradually. First, cooling to a temperature of about ambient temperature, and then cooling to a temperature of about -30°C, providing a frozen solution. Typically, the evaporation of the solvent is done at a temperature of about - 30°C. Preferably, evaporation of the solvent is done under reduced pressure (less than one atmosphere). Preferably, the solvent is removed at a pressure of about 1 mBar.

In one embodiment, the invention encompasses a composition containing Sunitinib base and racemic malic acid, characterized by data selected from a group consisting of: a PXRD pattern having any 5 peaks at positions selected from the group consisting of: 5.5, 8.3, 11.1, 14.2, 22.9, 23.8 and 26.8 ± 0.2 degrees 2-theta, a solid-state ¹³C NMR spectrum with signals at about 170.4, 157.2 and 115.5 ± 0.2 ppm, a solid-state ¹³C NMR spectrum having chemical shifts differences between the signal exhibiting the lowest chemical shift and another in the chemical shift range of 100 to 180 ppm of about 65.9, 52.7 and 11.0 ± 0.1 ppm, a PXRD pattern as depicted in Figure 33, a solid-state ¹³C NMR spectrum depicted in Figure 34, a solid-state ¹³C NMR spectrum depicted in Figure 35 and combination thereof. This composition can be designated as composition V-T.

Typically, the signal exhibiting the lowest chemical shift in the chemical shift area of 100 to 180 ppm is at about 104.5 ± 1 ppm.

In another embodiment, the invention encompasses a composition containing Sunitinib base and racemic malic acid, characterized by data selected from a group consisting of: a PXRD pattern as depicted in Figure 33, a solid-state ¹³C NMR spectrum depicted in Figure 34 and a solid-state ¹³C NMR spectrum depicted in Figure 35. This composition can be designated as composition V-T.

In a preferred embodiment, the invention encompasses a composition containing Sunitinib base and L-malic acid designated composition V-T characterized by a PXRD pattern having peaks at about 5.5 and 8.3 ± 0.2 degrees 2-theta, a PXRD pattern having any 3 peaks at positions selected from the group consisting of: 11.1, 14.2, 22.9, 23.8 and 26.8 ± 0.2 degrees 2-theta.

Composition V-T, can be further characterized by data selected from a group consisting of: a powder XRD pattern with peaks at about 5.5, 8.3, 11.1, 14.2 and 23.8 ± 0.2 degrees 2-theta; a powder XRD pattern with peaks at about 5.5, 8.3, 11.1, 22.9 and 26.8 ± 0.2 degrees 2-theta, a solid-state ¹³C NMR spectrum having signals at about 139.5 and 126.0 ± 0.2 ppm; and a solid-state ¹³C NMR spectrum having chemical shifts differences between the signal exhibiting the lowest chemical shift and another in the chemical shift range of 100 to 180 ppm of about 35.0 and 21.5 ± 0.1 ppm.

Composition V-T can be prepared by a process comprising lyophilizing an aqueous solution comprising Sunitinib base, racemic malic acid and water. Lyophilization is also known as freeze-drying.

In the lyophilization process, the said aqueous solution is subjected to a pressure of less than about one atmosphere, to remove the solvent.

Preferably, the aqueous solution is provided by a process comprising combining Sunitinib base, racemic malic acid and water and heating the said combination. Preferably, the mole ratio between Sunitinib base and L-malic acid is of about 1:2.

Preferably, freezing the solution is done gradually. First, cooling to a temperature of about ambient temperature, and then cooling to a temperature of about -30°C, providing a frozen solution. Typically, the evaporation of the solvent is done at a temperature of about - 30°C. Preferably, evaporation of the solvent is done under reduced pressure (less than one atmosphere). Preferably, the solvent is removed at a pressure of about 1 mBar.

The present invention further provides the above-described polymorphs of sunitinib malate, polymorphs of sunitinib hemi-L-malate or compositions containing sunitinib base and either L or racemic malic acid having less than about 15% by weight, of crystalline Sunitinib malate characterized by diffraction peaks at about 13.2 and 24.2 degrees two-theta, and more preferably, at about 13.2, 19.4, 24.2 and 25.5 degrees two-theta, designated form I.

Preferably, the above-described polymorphs of sunitinib malate, polymorphs of sunitinib hemi-L-malate or compositions containing sunitinib base and either L or racemic malic acid having less than about 10%, more preferably, less than about 5% by weight.

In yet another embodiment, the invention encompasses a process for preparing pharmaceutical composition comprising at least one of the above-described polymorphs of racemic sunitinib malate, sunitinib hemi-L-malate or of the above-described compositions containing Sunitinib base and either L or racemic malic acid, comprising combining at least one of the above-described polymorphs of racemic sunitinib malate, sunitinib hemi-L-malate or of the above-described compositions containing Sunitinib base and either L or racemic malic acid, and at least one pharmaceutically acceptable excipient.

In yet another embodiment, the invention encompasses a pharmaceutical composition comprising at least one of the above-described polymorphs of racemic sunitinib malate, sunitinib hemi-L-malate or of the above-described compositions containing Sunitinib base and L or racemic malic acid, prepared according to the processes disclosed herein and at least one pharmaceutically acceptable excipient.

In yet another embodiment, the invention encompasses a pharmaceutical composition comprising at least one of the above-described polymorphs of racemic sunitinib malate, sunitinib hemi-L-malate or of the above-described compositions containing Sunitinib base and L or racemic malic acid, and at least one pharmaceutically acceptable excipient.

In another embodiment, the invention encompasses a method of treating advanced renal cell carcinoma comprising administering to a patient in need thereof a pharmaceutical composition comprising at least one of the above-described polymorphs of racemic sunitinib malate, sunitinib hemi-L-malate or of the above compositions containing Sunitinib base and either L or racemic malic acid and at least one pharmaceutically acceptable excipient.

One embodiment of the invention provides the use of the above polymorphs of racemic Sunitinib malate, sunitinib hemi-L-malate or of the above-described compositions containing Sunitinib base and L or racemic malic acid of the present invention for the manufacture of a medicament for the treatment of gastrointenstinal stromal tumor or for the treatment of advanced renal cell

One embodiment of the invention provides the use of the above polymorphs of racemic Sunitinib malate, sunitinib hemi-L-malate or of the above-described compositions containing Sunitinib base and L or racemic malic acid of the present invention as a medicament for the treatment of gastrointenstinal stromal tumor or for the treatment of advanced renal cell carcinoma.

### Examples

### PXRD

XRD diffraction was performed on X-Ray powder diffractometer: Philips X'pert Pro powder diffractometer, CuKα radiation, λ = 1.5418 Å. X'Celerator detector active length (2 theta) = 2.122 mm, laboratory temperature 22-25°C. Zero background sample-holders. Prior to analysis the samples were gently ground by means of mortar and pestle in order to obtain a fine powder. The ground sample was adjusted into a cavity of the sample holder and the surface of the sample was smoothed by means of a cover glass.

### DSC

DSC measurements were performed on Differential Scanning Calorimeter DSC823e (Mettler Toledo). Al crucibles 40 µl with PIN were used for sample preparation. Typical weight of sample was 1 - 3 mg. Program: temperature range 25 - 300 °C, 10 °C/min.

A peak temperature or an onset temperature, were evaluated out of the DSC records. A peak temperature is defined as top of the DSC peak. An onset temperature is defined as the intersection point of the baseline before transition and the inflectional tangent.

### ¹³C NMR

The CP/MAS ¹³C NMR measurements were made at Bruker Avance 500 NMR US/WB spectrometer in 4-mm ZrO₂ rotor. Magic angle spinning (MAS) speed was 10 kHz. As used herein, the term ¹³C NMR chemical shifts" refers to the shifts measured under above specified conditions, however, these shifts can slightly differ instrument to instrument and can be shifted either upfield or downfield due to the different instrumental setup and calibration used. Nevertheless the sequence of individual peaks remains identical.

### Example 1: Preparation of Crystalline Form A of racemic Sunitinib Malate

Sunitinib base (form VII, 260 mg) was dissolved in anhydrous ethanol (23 ml) at 78°C. Racemic malic acid (95 mg) was added to the hot solution. The suspension was heated to 78°C for additional 5 min facilitating the complete dissolution of racemic malic acid. The solution was than allowed to cool to 15°C and to stand overnight. The crystals were recovered by filtration, washed with ethanol and dried in air.

### Example 2: Preparation of crystalline Form B of racemic Sunitinib malate

Sunitinib base (form VII, 130 mg) was dissolved in 1, 4-dioxane (3 ml) at 101°C. racemic malic acid (47 mg) dissolved in water (0.5 ml) was added to the hot solution. The solution was than allowed to cool to 15°C and to stand overnight. The crystals were recovered by filtration, washed with 1, 4-dioxane and dried in air.

### Example 3: Preparation of composition C containing sunitinib base and L-malic acid.

Sunitinib L-malate (form I, 200 mg) was dissolved in pyridine (2.25 ml) at 100 °C and heating for additional 30 minutes. Solution started to crystallize spontaneously upon cooling.

### Example 4: Preparation of Sunitinib hemi-L-malate Form E

Sunitinib base (Form II, 300 mg), L-malic acid (50 mg = molar ratio sunitinib: L-malic acid = 1: 0.5), and water (3 ml) were heated to complete dissolution (about 3 min). The solution was frozen and lyophilized at 1 mBar 20 °C.

### Example 5: Preparation of composition F containing sunitinib base and L-malic acid.

Sunitinib base (form X, containing about 15 % of water as determined by KF, 300 mg), L-malic acid (101 mg), t-BME (10 ml), ultrasound 30 min, 20 °C, filtered, dried in air.

### Example 6: Preparation of composition L containing sunitinib base and L-malic acid.

Sunitinib base (form VII, 900 mg), L-malic acid (305 mg), and water (9 ml) were heated to complete dissolution (about 3 min). The solution was frozen and lyophilized at 1 mbar 20 °C.

### Example 7: Preparation of composition I containing sunitinib base and L-malic acid.

To sunitinib base (form VII, 300 mg) and L-malic acid (110 mg) was added water (3 ml) and the slurry was heated to 100 °C facilitating complete dissolution. The sample was frozen, lyophilized at 1 mBar at 25 °C, and annealed at 38 °C for 1 h.

### Example 8: Preparation of composition J containing sunitinib base and L-malic acid.

To sunitinib base (form VII, 300 mg) and L-malic acid (150 mg), molar ratio 1:1.5 was added water (3 ml) and the slurry was heated to 100 °C facilitating complete dissolution. The sample was frozen, lyophilized at 1 mBar at 25°C, and annealed at 38°C for 1 h.

### Example 9: Preparation of composition K containing sunitinib base and L-malic acid.

To sunitinib base (form VII, 300 mg) and L-malic acid (200 mg) was added water (3 ml) and the slurry was heated to 100°C facilitating complete dissolution. The sample was frozen, lyophilized at 1 mbar at 25°C, and annealed at 38°C for 1 h.

### Example 10: Preparation of composition H containing sunitinib base and L-malic acid.

To sunitinib base (form VII, 300 mg) and L-malic acid (101 mg) was added TBME 10 ml) and the slurry was heated 10 min to reflux temperature facilitating formation of the titled composition (368 mg).

### Example 11: Preparation of composition G containing sunitinib base and L-malic acid.

Sunitinib L-malate (form 1, 500 mg) was slurred in 10 ml water, and then the slurry was lyophilized.

### Example 12: Preparation of composition N containing sunitinib base and L-malic acid.

Sunitinib L-malate (form 1, 500 mg) was dissolved in 50 ml dioxane containing 5% of water, and then the solution was lyophilized.

### Example 13: Preparation of composition O containing sunitinib base and L-malic acid.

Sunitinib base (form X, dried 70°C, 2 h, 1 mBar, 150 mg), L-malic acid (50 mg in 3 ml of water, 1:1 molar), THF (8 ml), boiling to dissolution, allowed to evaporate at 20°C to dryness in an open baker.

### Example 14: Preparation of composition P containing sunitinib base and L-malic acid.

Sunitinib base (form X, dried 70°C, 2 h, 1 mBar, 150 mg), L-malic acid (50 mg in 1.5 ml of water, 1:1 molar), dioxolane (5 ml), boiling to dissolution, allowed to evaporate at 20°C to dryness in an open baker.

### Example 15: Preparation of composition Q containing sunitinib base and L-malic acid.

Sunitinib base (form X, dried 70°C, 2 h, 1 mBar, 150 mg) L-malic acid (50 mg in 1.5 ml of water, 1:1 molar), dioxane (5 ml), boiling to dissolution, allowed to evaporate at 20°C to dryness in an open baker.

### Example 16: Preparation of composition R containing sunitinib base and L-malic acid.

1 g of sunitinib base (form D) was charged in a vessel with 0,34 g of L-malic acid and 25 ml of DMSO. The system was stirred and after a few minutes a solution was observed. The solution, after filtration, was lyophilized. 1,2 g of solid was obtained.

### Example 17: Preparation of sunitinib hemi-L-malate form U

Sunitinib base (Form D, 300 mg) was dissolved in pyridine (3 ml) by heating and than the solution was quickly frozen to about 150 K by placing the flask in liquid nitrogen. Then L-malic acid (110 mg in 1 ml of methanol) was added and the mixture was allowed to heat up to -30°C in refrigerator and allowed to stand 5 days. Then the suspension was filtered, washed with t-butyl methyl ether (5 ml) and dried in air.

### Example 19: Preparation of composition M containing sunitinib base and L-malic acid. Sunitinib L-malate (composition G) was heated at 80 °C, 1 mBar for 2 hours.

### Example 20: Preparation of composition V-A containing Sunitinib base and L-malic acid

Sunitinib base (form VIII) and L-malic acid having a PXRD pattern with peaks at about 24.4 and 29.4 ± 0.2 degrees 2-theta and any 3 peaks selected from a list consisting of: 19.3, 24.4, 29.4, 29.7and 30.2 ± 0.2 degrees 2-theta and having a PXRD pattern as depicted in Figure 6 were ground separately in mortar by means of pestle to get a fine powder. Then 60 mg of the ground Sunitinib base and 20 mg of the ground L-malic acid (corresponds to molar ratio 1:1) were weighed into a vial and homogenized by shaking the vial.

### Example 21: Preparation of composition V-B containing Sunitinib base and L-malic acid

The composition V-A prepared according to the procedure described in the Example 20 was kept in a closed vial and exposed to temperature about 80 °C for 4 hours. Stability of the composition during the heating was confirmed by mass spectrometry that proved presence of Sunitinib base and L-malic acid in the sample after the heating. No decomposition products were detected by the method.

### Example 22: Preparation of composition V-C containing Sunitinib base and L-malic acid

Sunitinib base (form VIII,) and L-malic acid having a PXRD pattern with peaks at about 24.4 and 29.4 ± 0.2 degrees 2-theta and any 3 peaks selected from a list consisting of: 19.3, 24.4, 29.4, 29.7and 30.2 ± 0.2 degrees 2-theta and having a PXRD pattern as depicted in Figure 6 were ground separately in mortar by means of pestle to get a fine powder. Then 280 mg of the ground Sunitinib base and 70 mg of the ground L-malic acid (corresponds to molar ratio 1:1) were weighed into a vial and homogenized by shaking the vial. About 10 mg of the mixture was then placed into another vial, 10 microliters of water was added and the vial was closed. The slurry was mixed by shaking the vial and left for 3 days at room temperature.

### Example 23: Preparation of composition V-S containing sunitinib and L-malic acid

Sunitinib base (Form X) having a PXRD pattern with any 5 peaks selected from a group consisting of 3.9, 7.8, 9.1, 10.1, 11.6, 14.3, 15.9, 18.2, 20.4, 23.1, 23.9 and 27.0 deg ± 0.2 degrees 2-theta (442 mg), L-malic acid (297 mg, mol 1:2) and water (10 ml) were heated to complete dissolution. The solution was allowed to cool to ambient temperature and frozen to - 30°C. Then the lyofilization was carried out at 1 mBar providing a solid.

### Example 24: Preparation of composition V-T containing Sunitinib and racemic malic acid

Sunitinib base (Form X) having a PXRD pattern with any 5 peaks selected from a group consisting of 3.9, 7.8, 9.1, 10.1, 11.6, 14.3, 15.9, 18.2, 20.4, 23.1, 23.9 and 27.0 deg ± 0.2 degrees 2-theta (442 mg), racemic malic acid 297 mg, mol 1:2) and water (10 ml) were heated to complete dissolution. The solution was allowed to cool to ambient temperature and frozen to - 30°C. Then the lyofilization was carried out at 1 mBar providing a solid.

### Example 25: Preparation of Sunitinib base form II:

Sunitinib base was dissolved in water (250 g) at about 40° by adjusting the pH to 1.5 with 1 N HCl. The solution was extracted with methyl isobutyl ketone (100 g), the phases were separated and to the aqueous phase dimethylacetamide (20 g) was added. Under strong stirring, the solution was adjusted to pH 8.5 by addition of 25% ammonium hydroxide solution. After one hour the suspension was filtered and the cake was rinsed with 200 g of water. The product was dried at 70° under vacuum overnight to give 12.6 g (56%) of Sunitinib base Form II.

### Example 26: Preparation of Sunitinib base form VIII:

Sunitinib base, obtained by reaction of Sunitinib activated carboxylic acid derivative with excess of N, N'-diethylaminoethylamine in 2-Methyltetrahydrofuran, was dissolved in 15 volumes of water (150ml for 10g of sunitinib base) at pH 2 (obtained by addition of HCl 1 M) at 70°C. The mixture was then cooled to 25°C and precipitated with the addition of ammonia 30% to pH 8.5 at 25°C, stirred for one hour and filtered at the same temperature, washed with water and dried in oven under vacuum for 16 hours at 60°C.

### Example 27: Preparation of Sunitinib base form X:

20g of Sunitinib Base are dissolved with 570g of water and 190g of HCl 1 M. The solution was washed with methyl isobutyl ketone and then precipitated with ammonia 30% in water to pH 9, the product was filtered and dried in oven under vacuum at 60°C.
The sample contains 15% water by KF.

### Example 28: Preparation of Sunitinib base Form D:

Sunitinib base (Form II, 300 mg), L-malic acid (101 mg) and toluene (10 ml) were heated to reflux 10 min. The suspension was allowed to cool to room temperature, filtered, washed with n-hexane and dried in air.

### Example 29: Preparation of Sunitinib base form VII:

5g of Sunitinib Base, obtained by reaction of Sunitinib activated carboxylic acid derivative with excess of N,N'-diethylaminoethylamine in tetrahydrofuran, were dissolved with 150g of water and 50g of HCl 1 M. The solution was washed with methyl isobutyl ketone at 50°C and then precipitated with ammonia 30% in water to pH 9, the product was filtered and dried in oven under vacuum at 60°C for 16 hours.

### Example 30: Conversion of Sunitinib to Sunitinib Malate (according to Example 1, Preparation A of U.S. publication No. 2003/0069298):

Preparation of the Anhydrous Crystal Form I of the L-Malic Acid Salt of N-[2-(Diethylamino) ethyl]-5-[(5fluoro-1, 2-dihydro-2-oxo-3H-indol-- 3-ylidene) methyl]-2, 4-dimethyl-1 H-pyrrole-3-carboxamide.

### Preparation A:

N-[2-(Diethylamino)ethyl]-5-[(5-fluoro-1,2-dihydro-2- -oxo-3H-indol-3-ylidene)methyl]-2,4-dimethyl-1 H-pyrrole-3-carboxamide (130 mg, 0.326 mmol) was added to 20 mL methanol, and the mixture was stirred. L-malic acid (47.2 mg, 0.352 mmol) was added, resulting in rapid dissolution of all the solids. The methanol was removed under reduced pressure to produce a poorly crystalline orange solid. Acetonitrile (5 mL) was added, and the slurry was stirred and heated for about 10 minutes. Stirring was continued while the slurry was allowed to cool to room temperature. The crystals were filtered and dried, resulting in 149 mg of solids (86% yield).

### Example 31: Preparation of sunitinib base via 5-(5-fluoro-2-oxo-1, 2-dihydro-indol-3Z-ylidenemethyl)-2, 4-dimethyl-1 H-pyrrole-3-carbonyl chloride

31.2 g of 5-(5-fluoro-2-oxo-1, 2-dihydro-indol-3Z-ylidenemethyl)-2,4-dimethyl-1 H-pyrrole-3-carboxylic acid, were refluxed under stirring for 4 hours in one liter flask with 310 g of toluene, 15 g of thionyl chloride and 1 g of dimethylformamide.

The stirred suspension was cooled at room temperature for 2 hours and filtered; the cake was washed with 50 g of toluene and dried at 50° under vacuum overnight.

Yield was 32.4 g (97.8%) of a composition corresponding by NMR and MS to the expected structure.

20 g of diethylendiamine were dissolved in one liter flask with 300 g of tetrahydrofuran; about 200 g of solvent were distilled away at 50° under vacuum.

20 g of 5-(5-fluoro-2-oxo-1,2-dihydro-indol-3Z-ylidenemethyl)-2,4-dimethyl-1H-pyrrole-3-carbonyl chloride, prepared as above, were added under stirring and solution obtained was left for one hour to react without more heating.

300 g of water were added and the suspension was evaporated at 50° under vacuum to eliminate most of organic solvent.

After stirring 2 hours at room temperature the suspension was filtered, washed with 100 g of water and dried at 50° under vacuum overnight, obtaining 23.5 g of crude Sunitinib.

### Purification

Crude material was dissolved with 560 g of water and 190 g of 1 M Hydrochloric acid, extracted with 200 g of methyl-isobutyl ketone.

Clarified aqueous phase was basified under stirring with concentrated aqueous ammonia to pH 8.5 and after 2 hours the suspension was filtered and crystals were washed with 100 g of water. Product was dried at 50° under vacuum overnight obtaining 20.5 (82% yield) of sunitinib.

### Example 31: Preparation of sunitinib base via Sunitinib carboxylic acid derivative

In a 500 ml reactor, 15.0 g. of Sunitinib carboxylic acid derivative were suspended into 300ml of toluene (ratio 20/1.0 v/w starting material) under vigorous stirring at room temperature.

0.755 g of dimethylformamide (ratio 0.2/1.0 w/w) were added to the mixture.

The temperature was set at 70°C and at this temperature; 5.1 g of thionyl chloride (ratio 1.4/1.0 w/w) were dropped in a range of sixty minutes.

The reaction was kept at 70°C for 7 hours under stirring.

Then 140 ml of solvent were distilled to remove excess of thionyl chloride from the suspension and the reaction filtered on gooch P3 washing with 3v/w of toluene. The wet solid (sunitinib acyl chloride derivative) was re-loaded into the reactor and 300ml Methyl-tetrahydrofuran was loaded and stirred. Then the reaction mixture was heated to 70°C and 6.35g of 2-diethylamino-ethylamine (ratio 1.1/1.0 w/w starting material) were dropped in five minutes at 70°C. After one hour the reaction was completed and 150 ml of water and HCl 2N until pH 2 were added to the suspension.

A following filtration of the mixture using a decalite pad was done to obtain a clarified phase. The two phases were separated at 50°C and the organic phase discarded. The aqueous phase was washed once more with 300ml of Methyl-tetrahydrofuran at 50°C under stirring. The two phases were separated again and the organic phase discarded.

The aqueous phase was then basified to pH 8.5 with 5% ammonia solution at 50°C.

After one hour stirring, the suspension was filtered on gooch P3 and the wet solid dried at 60°C under vacuum overnight.

15.9 g. of sunitinib base were obtained with a purity of NLT 99.5% by HPLC.

### Example 33: preparation of Sunitinib malate form I:

Sunitinib base (1500 mg) was dissolved in ethanol (62 ml) at 78°C and L-malic acid (525 mg in 3 ml of water) was added. Crystals were formed overnight, filtered and dried in air.

The present invention also includes the following numbered embodiments:
1. Sunitinib hemi-L-malate.
2. The Sunitinib hemi-L-malate of embodiment 1, wherein the Sunitinib hemi-L-malate is solid.
3. The Sunitinib hemi-L-malate of embodiment 1 or 2, wherein the Sunitinib hemi-L-malate is crystalline.
4. A crystalline form of sunitinib hemi-L-malate characterized by data selected from the group consisting of a PXRD pattern having any 5 peaks at positions selected from the group consisting of: 3.7, 6.8, 10.3, 11.3, 11.9, 14.2, 15.1, 15.9, 25.9 and 26.6 ± 0.2 degrees 2-theta, a PXRD pattern as depicted in Figure 4, a PXRD pattern as depicted in Figure 4a and combination thereof.
5. The crystalline form of sunitinib hemi-L-malate of embodiment 4, characterized by a PXRD pattern having any 5 peaks at positions selected from the group consisting of: 3.7, 6.8, 10.3, 11.3, 11.9, 14.2, 15.1, 15.9, 25.9 and 26.6 ± 0.2 degrees 2-theta.
6. The crystalline form of sunitinib hemi-L-malate of embodiment 4 or 5, characterized by data selected from the group consisting of a PXRD pattern as depicted in Figure 4, a PXRD pattern as depicted in Figure 4a and combination thereof.
7. The crystalline form of sunitinib hemi-L-malate of embodiment 4 or 5, characterized by a PXRD pattern having peaks at about 6.8 and 11.3 ± 0.2 degrees 2-theta and any 3 peaks at positions selected from the group consisting of: 3.7, 11.9, 15.1, 25.9 and 26.6 ± 0.2 degrees 2-theta.
8. The crystalline form of sunitinib hemi-L-malate of embodiment 7, further characterized by a PXRD pattern having peaks at about 3.7, 6.8, 11.3, 11.9 and 25.9 ± 0.2 degrees 2-theta.
9. A crystalline form of sunitinib hemi-L-malate characterized by data selected from the group consisting of: a PXRD pattern any 5 peaks at positions selected from the group consisting of: 5.8, 9.6, 13.9, 15.9, 22.7, 26.6 and 28.7 ± 0.2 degrees 2-theta, a solid-state ¹³C NMR spectrum with signals at about 169.0, 136.0 and 119.5 ± 0.2 ppm, a solid-state ¹³C NMR spectrum having chemical shifts differences between the signal exhibiting the lowest chemical shift and another in the chemical shift range of 100 to 180 ppm of about 66.3, 33.3, 16.8 ± 0.1 ppm, a PXRD pattern as depicted in Figure 18; a ¹³C NMR spectrum depicted in Figure 19, and a solid-state ¹³C NMR spectrum depicted in Figure 20 and combination thereof.
10. The crystalline form of sunitinib hemi-L-malate of embodiment 9, characterized by a PXRD pattern any 5 peaks at positions selected from the group consisting of: 5.8, 9.6, 13.9, 15.9, 22.7, 26.6 and 28.7 ± 0.2 degrees 2-theta.
11. The crystalline form of sunitinib hemi-L-malate of embodiment 9 or 10, characterized by a solid-state ¹³C NMR spectrum with signals at about 169.0, 136.0 and 119.5 ± 0.2 ppm.
12. The crystalline form of sunitinib hemi-L-malate of any of embodiments 9 to 11, characterized by a solid-state ¹³C NMR spectrum having chemical shifts differences between the signal exhibiting the lowest chemical shift and another in the chemical shift range of 100 to 180 ppm of about 66.3, 33.3, 16.8 ± 0.1 ppm
13. The crystalline form of sunitinib hemi-L-malate of any of embodiments 9 to 12, characterized by a PXRD pattern as depicted in Figure 18.
14. The crystalline form of sunitinib hemi-L-malate of any of embodiments 9 to 13, characterized by data selected from a group consisting of: a ¹³C NMR spectrum depicted in Figure 19, and a solid-state ¹³C NMR spectrum depicted in Figure 20 and combination thereof.
15. The crystalline form of sunitinib hemi-L-malate of any of embodiments 9 to 12, characterized by a PXRD pattern having peaks at about 5.8 and 22.7 ± 0.2 degrees 2-theta and any 3 peaks at positions selected from the group consisting of: 9.6, 13.9, 15.9, 26.6 and 28.7 ± 0.2 degrees 2-theta
16. The crystalline form of sunitinib hemi-L-malate of embodiment 15, further characterized by a powder XRD pattern with peaks at about 5.8, 13.9, 22.7, 26.6 and 28.7 ± 0.2 degrees 2-theta
17. The crystalline form of sunitinib hemi-L-malate of embodiment 15, further characterized by a powder XRD pattern with peaks at about 5.8, 9.6, 22.7, 26.6 and 28.7 ± 0.2 degrees 2-theta.
18. The crystalline form of sunitinib hemi-L-malate of any of embodiments 9 to 17, further characterized by a thermogram depicted in Figure 21 and having DSC peak at about 218.8 ± 2 °C.
19. The crystalline form of sunitinib hemi-L-malate of any of embodiments 9 to 18, further characterized by a solid-state ¹³C NMR spectrum having signals at about 156.6 and 117.5 ± 0.2 ppm.
20. The crystalline form of sunitinib hemi-L-malate of any of embodiments 9 to 19, further characterized by and a solid-state ¹³C NMR spectrum having chemical shifts differences between the signal exhibiting the lowest chemical shift and another in the chemical shift range of 100 to 180 ppm of about 53.9 and 14.8 ± 0.1 ppm.
21. Racemic sunitinib malate.
22. The racemic Sunitinib malate of embodiment 21, wherein the racemic Sunitinib malate is solid.
23. The racemic Sunitinib malate of embodiment 21 or 22, wherein it is crystalline.
24. A racemic sunitinib malate characterized by data selected from the group consisting of a PXRD pattern having any 5 peaks at positions selected from the group consisting of: 5.7, 8.0, 9.3, 12.5, 14.7, 15.4, 18.3, 21.5, 24.7 and 27.4 ± 0.2 degrees 2-theta, a PXRD pattern as depicted in Figure 1 and combination thereof.
25. The racemic sunitinib malate of embodiment 24, characterized by a PXRD pattern having any 5 peaks at positions selected from the group consisting of: 5.7, 8.0, 9.3, 12.5, 14.7, 15.4, 18.3, 21.5, 24.7 and 27.4 ± 0.2 degrees 2-theta.
26. The racemic sunitinib malate of embodiment 24 or 25, characterized by a PXRD pattern as depicted in Figure 1
27. The racemic sunitinib malate of embodiment 24 or 25, characterized by a PXRD pattern having peaks at about 5.7 and 8.0 ± 0.2 degrees 2-theta and any 3 peaks at positions selected from the group consisting of 9.3, 12.5, 15.4, 18.3, 21.5, 24.7 and 27.4 ± 0.2 degrees 2-theta.
28. The racemic sunitinib malate of embodiment 27, characterized by a PXRD pattern having peaks at about 5.7, 8.0, 12.5, 14.7 and 18.3 ± 0.2 degrees 2-theta.
29. The racemic sunitinib malate of embodiment 27, characterized by a PXRD pattern having peaks at about 5.7, 8.0, 9.3, 24.7 and 18.3 ± 0.2 degrees 2-theta.
30. The racemic sunitinib malate of embodiment 27, characterized by and a PXRD pattern having a single peak at about 27.4 ± 0.2 degrees 2-theta.
31. A racemic sunitinib malate characterized by data selected from the group consisting of a PXRD pattern having any 5 peaks at positions selected from the group consisting of: 15.2, 16.1, 22.1, 23.5, 24.5, 27.2 and 27.5 ± 0.2 degrees 2-theta, a PXRD pattern as depicted in Figure 2 and combination thereof.
32. The racemic sunitinib malate of embodiment 31, characterized by a PXRD pattern having any 5 peaks at positions selected from the group consisting of: 15.2, 16.1, 22.1, 23.5, 24.5, 27.2 and 27.5 ± 0.2 degrees 2-theta.
33. The racemic sunitinib malate of embodiment 31 or 32, characterized by a PXRD pattern as depicted in Figure 2.
34. The racemic sunitinib malate of embodiment 31 or 32, characterized by a PXRD pattern having peaks at about 7.5 and 22.1 ± 0.2 degrees 2-theta and any 3 peaks at positions selected from the group consisting of 15.2, 16.1, 23.5, 24.5, 27.2 and 27.5 ± 0.2 degrees 2-theta.
35. The racemic sunitinib malate of embodiment 34, further characterized by a PXRD pattern having peaks at about 15.2, 16.1, 22.1, 27.2 and 27.5 ± 0.2 degrees 2-theta.
36. The racemic sunitinib malate of embodiment 34, further characterized by a PXRD pattern having a double peak at about 27.2 and 27.5 ± 0.2 degrees 2-theta.
37. Use of the racemic sunitinib malate of any of embodiments 21 to 36 or the sunitinib hemi-L-malate of any of embodiments 1 to 20 for the manufacture of a medicament for the treatment of gastrointenstinal stromal tumor or for the treatment of advanced renal cell carcinoma.
38. The racemic sunitinib malate as defined in any of embodiments 21 to 36 or the sunitinib hemi-L-malate as defined in any of embodiments 1 to 20 for use as a medicament for the treatment of gastrointenstinal stromal tumor or for the treatment of advanced renal cell carcinoma.
39. A pharmaceutical composition comprising at least one of the racemic sunitinib malate of any of embodiments 21 to 36 or the sunitinib hemi-L-malate of any of the embodiments 1 to 20 and at least one pharmaceutically acceptable excipient.
40. A process for preparing a pharmaceutical composition comprising at least one of the racemic sunitinib malate of any of embodiments 21 to 36 and the sunitinib hemi-L-malate of any of embodiments 1 to 20, comprising combining said at least one of the racemic sunitinib malate and the sunitinib hemi-L-malate and at least one pharmaceutically acceptable excipient.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. Racemic sunitinib malate.

2. The racemic Sunitinib malate of claim 1, wherein the racemic Sunitinib malate is solid, preferably, wherein it is crystalline.

3. The racemic sunitinib malate of claim 1 or claim 2, **characterized by** data selected from the group consisting of a PXRD pattern having any 5 peaks at positions selected from the group consisting of: 5.7, 8.0, 9.3, 12.5, 14.7, 15.4, 18.3, 21.5, 24.7 and 27.4 ± 0.2 degrees 2-theta, a PXRD pattern as depicted in Figure 1 and combinations thereof.

4. The racemic sunitinib malate of claim 3, **characterized by** a PXRD pattern having peaks at about 5.7 and 8.0 ± 0.2 degrees 2-theta and any 3 peaks at positions selected from the group consisting of 9.3, 12.5, 15.4, 18.3, 21.5, 24.7 and 27.4 ± 0.2 degrees 2-theta.

5. The racemic sunitinib malate of claim 4, **characterized by** a PXRD pattern having peaks at about 5.7, 8.0, 12.5, 14.7 and 18.3 ± 0.2 degrees 2-theta.

6. The racemic sunitinib malate of claim 4, **characterized by** a PXRD pattern having peaks at about 5.7, 8.0, 9.3, 24.7 and 18.3 ± 0.2 degrees 2-theta.

7. The racemic sunitinib malate of claim 4, **characterized by** and a PXRD pattern having a single peak at about 27.4 ± 0.2 degrees 2-theta.

8. The racemic sunitinib malate of claim 1 or claim 2, **characterized by** data selected from the group consisting of a PXRD pattern having any 5 peaks at positions selected from the group consisting of: 15.2, 16.1, 22.1, 23.5, 24.5, 27.2 and 27.5 ± 0.2 degrees 2-theta, a PXRD pattern as depicted in Figure 2 and combinations thereof.

9. The racemic sunitinib malate of claim 8, **characterized by** a PXRD pattern having any 5 peaks at positions selected from the group consisting of: 15.2, 16.1, 22.1, 23.5, 24.5, 27.2 and 27.5 ± 0.2 degrees 2-theta.

10. The racemic sunitinib malate of claim 8 or 9, **characterized by** a PXRD pattern having peaks at about 7.5 and 22.1 ± 0.2 degrees 2-theta and any 3 peaks at positions selected from the group consisting of 15.2, 16.1, 23.5, 24.5, 27.2 and 27.5 ± 0.2 degrees 2-theta.

11. The racemic sunitinib malate of claim 10, further **characterized by** a PXRD pattern having peaks at about 15.2, 16.1, 22.1, 27.2 and 27.5 ± 0.2 degrees 2-theta.

12. The racemic sunitinib malate of claim 10, further **characterized by** a PXRD pattern having a double peak at about 27.2 and 27.5 ± 0.2 degrees 2-theta.

13. The racemic sunitinib malate of any of claims 1 to 12 for use in the treatment of gastrointenstinal stromal tumor or in the treatment of advanced renal cell carcinoma.

14. A pharmaceutical composition comprising the racemic sunitinib malate of any of claims 1 to 12 and at least one pharmaceutically acceptable excipient.

15. A process for preparing a pharmaceutical composition comprising the racemic sunitinib malate of any of claims 1 to 12, comprising combining said racemic sunitinib malate and at least one pharmaceutically acceptable excipient.
